# EUROPEAN PATENT APPLICATION

(11) **EP 4 775 178 A1**
(43) Date of publication of application: **15.07.2026**
(21) Application number: 24862106.2
(22) Date of filing: 06.09.2024
(51) Int. Cl.: A61F 2/07

(54) **COVERED STENT, STENT SYSTEM AND USAGE**

(30) Priority: 08.09.2023 CN 202311161041
(71) Applicant: Hangzhou Endonom Medtech Co., Ltd, Hangzhou, Zhejiang 310051 (CN)
(72) Inventor: WANG, Yongsheng, Hangzhou, Zhejiang 310051 (CN); LI, Anwei, Hangzhou, Zhejiang 310051 (CN); WANG, Dong, Hangzhou, Zhejiang 310051 (CN)
(74) Representative: Mathys & Squire
(86) International application number: PCT/CN2024/117613
(87) International publication number: WO 2025/051268

(57) **Abstract**

A stent graft, a stent system, and an application method are provided. The stent graft includes a main stent (10) and a connecting stent (20). The main stent (10) is tubular. The connecting stent (20) is tubular and defines a first opening (201) and a second opening (202). The first opening (201) and the second opening (202) are defined at two ends of the connecting stent in an axial direction of the connecting stent. Either of the first opening (201) and the second opening (202) is fixed to the main stent (10). An inner cavity of the connecting stent (20) is in communication with a lumen of the connecting stent (10). The connecting stent (20) has a first configuration and a second configuration, and is switchable between the first configuration and the second configuration. An axial length of the connecting stent (20) in the first configuration is larger than an axial length of the connecting stent in the second configuration. In this way, the connecting stent (20) can extend out of the main stent (10) to form an extended branch, thereby reducing a lumen loss rate of the main stent. The connecting stent (20) can also retract towards the lumen of the main stent (20) to form an embedded branch, so as to reconstruct some vessels in which there is no sufficient space at a branch vessel to be compatible with an extended branch structure.

## Description

### CROSS-REFERENCE TO RELATED APPLICATION(S)

This application claims priority to Chinese Patent Application No. 202311161041.4, filed with the China National Intellectual Property Administration on September 08, 2023 and entitled "STENT GRAFT, STENT SYSTEM, AND APPLICATION METHOD", the entire disclosure of which is hereby incorporated by reference.

### TECHNICAL FIELD

This disclosure relates to the field of medical device technology, and in particular, to a stent graft, a stent system, and an application method.

### BACKGROUND

Thoracic endovascular aortic repair (TEVAR) or endovascular aneurysm repair (EVAR) refers to a therapeutic method. In this therapeutic method, a stent artificial endovascular graft is used, and is radially compressed and assembled into an interventional sheath tube, generally via a small incision in an iliofemoral artery as an access route. Using interventional therapy techniques, the interventional sheath tube is advanced through an aortic lumen to a predetermined position of an aorta, and then the interventional sheath tube is withdrawn to release the endovascular graft. Within the aortic lumen, the endovascular graft seals or excludes a dissection tear or an aneurysmal sac, thereby establishing blood flow through an aortic true lumen and a branch artery, while closing or reducing blood flow in the aortic dissection or the aneurysmal sac. Compared with the method of open surgery, TEVAR or EVAR has the advantages of less trauma, low perioperative mortality, rapid recovery, etc. In recent years, more and more doctors and patients choose TEVAR or EVAR for treatment. With the successful application of branched stent technology in visceral endovascular repair, the gradual development of branched stent technology in TEVAR or EVAR treatment has changed the pattern of arterial disease treatment and expanded the surgical indications of whole endovascular repair technology.

### SUMMARY

In some implementations, the present disclosure provides a stent graft. The stent graft includes a main stent and a connecting stent. The main stent defines two openings at two ends of the main stent in an axial direction of the main stent and a lumen extending axially between the two openings. The main stent defines at least one fenestration on a sidewall of the main stent. The connecting stent defines a first opening, a second opening, and an inner cavity extending between the first opening and the second opening. The first opening and the second opening are defined at two ends of the connecting stent in an axial direction of the connecting stent. Either of the first opening and the second opening is fixed to one of the at least one fenestration of the main stent, to enable the inner cavity of the connecting stent to be in communication with the lumen of the main stent through the fenestration. The connecting stent has a first configuration and a second configuration. The connecting stent is switchable between the first configuration and the second configuration. An axial length of the connecting stent in the first configuration is larger than an axial length of the connecting stent in the second configuration.

### BRIEF DESCRIPTION OF THE DRAWINGS

FIG. 1 is a schematic structural view of a connecting stent in an extended state on a stent system in a first embodiment of the present disclosure.
FIG. 2 is a schematic structural view of a connecting stent in the extended state on a stent graft illustrated in FIG. 1.
FIG. 3 is a schematic structural view of a connecting stent in a retracted state on the stent system illustrated in FIG. 1.
FIG. 4 is a schematic structural view of the connecting stent in the retracted state on a stent graft illustrated in FIG. 3.
FIG. 5 is a partial enlarged view of the stent graft illustrated in FIG. 2 at Circle A.
FIG. 6 is a partial enlarged view of the stent graft illustrated in FIG. 4 at Circle B.
FIG. 7 is a schematic structural view of the stent graft illustrated in FIG. 5 in other implementations.
FIG. 8 is a schematic structural view of the stent graft illustrated in FIG. 6 in other implementations.
FIG. 9 is a schematic structural view of a main stent in some implementations.
FIG. 10 is an exemplary application scenario diagram of a stent graft in the first embodiment.
FIG. 11 is a schematic structural view of a stent graft in a second embodiment.
FIG. 12 is a schematic structural view of the stent graft in FIG. 11 from another perspective.
FIG. 13 is a schematic structural view of a stent graft in some implementations.
FIG. 14 is a schematic structural view of the stent graft in FIG. 13 from another perspective.
FIG. 15 is an exemplary application scenario diagram of a stent graft in the second embodiment.
FIG. 16 is a schematic structural view of a stent graft in a third embodiment.
FIG. 17 is a schematic structural view of the stent graft in FIG. 16 from another perspective.
FIG. 18 is an exemplary application scenario diagram of a stent graft in the third embodiment.
FIG. 19 is a schematic structural view of a stent graft in a fourth embodiment.
FIG. 20 is a partial enlarged schematic view of FIG. 19 at Circle C.
FIG. 21 is a schematic structural view of the stent graft illustrated in FIG. 19 from another perspective.
FIG. 22 is a schematic view of rotation of a first stent in the stent graft in the fourth embodiment in any direction.
FIG. 23 is a schematic structural view of the first stent in the retracted state in the stent graft in the fourth embodiment.
FIG. 24 is a schematic structural view of a first stent in a stent graft in other implementations.
FIG. 25 is an exemplary application scenario diagram of a stent graft in the fourth embodiment.
FIG. 26 is a schematic structural view of a stent system in a fifth embodiment (schematic view illustrating at least partial release of a proximal end of the stent graft).

Reference signs in the accompanying drawings are described as follows: 1, stent graft; 10, main stent; 101, main covering membrane; 102, main support frame; 1021, support rod; 1022, crest; 1023, trough; 1024, surrounding angle; 1025, wall apposition angle; 1026, sealing angle; 11, fenestration; 111, first fenestration; 112, second fenestration; 113, third fenestration; 12, support ring; 121, first support ring; 122, second support ring; 123, third support ring; 13, first support segment; 14, recessed segment; 141, avoidance segment; 142, flared portion; 15, second support segment; 20, connecting stent; 201, first opening; 202, second opening; 203, first segment; 204, second segment; 205, neck; 206, connecting member; 207, connecting covering membrane; 208, support member; 209, radiopaque structure; 2091, first annular radiopaque member; 2092, second annular radiopaque member; 210, third opening; 30, anchor stent; 31, first stent; 32, second stent; 40, reinforcing semi-annular structure; 2, branch stent; 21, branch covering membrane; 22, branch support frame; 3, aortic arch; 4, branch vessel; 401, brachiocephalic artery; 402, left common carotid artery; 403, left subclavian artery; 5, interventional sheath tube; 6, guide head; 7, interventional sheath core.

### DETAILED DESCRIPTION

For a stent graft in some implementations, the stent graft includes a main stent and a connecting stent. The main stent defines two openings at two ends of the main stent in an axial direction of the main stent, and a lumen extending axially between the two openings. The main stent defines at least one fenestration on a sidewall of the main stent. The connecting stent defines a first opening, a second opening, and an inner cavity extending between the first opening and the second opening. The first opening and the second opening are defined at two ends of the connecting stent in an axial direction of the connecting stent. The connecting stent is fixed to the at least one fenestration of the main stent through one end of the connecting stent where the second opening is located, to enable the connecting stent to be in communication with the main stent. The connecting stent has a first configuration and a second configuration. The connecting stent is switchable between the first configuration and the second configuration. An axial length of the connecting stent in the first configuration is larger than an axial length of the connecting stent in the second configuration.

For the stent graft in some implementations, the connecting stent at least partially consists of a corrugated tube in the axial direction of the connecting stent.

For the stent graft in some implementations, the connecting stent includes a first segment and a second segment that is fixed to and in communication with the first segment. At least one of the first segment of the connecting stent or the second segment of the connecting stent consists of the corrugated tube in the axial direction of the connecting stent.

For the stent graft in some implementations, the connecting stent in the first configuration is located outside the main stent.

For the stent graft in some implementations, the connecting stent in the second configuration is at least partially located in the lumen of the main stent.

For the stent graft in some implementations, the connecting stent includes a first segment and a second segment that is fixed to and in communication with the first segment. When the connecting stent is in the first configuration, the first segment and the second segment are arranged in the axial direction of the connecting stent, a neck of the connecting stent is formed at a connection of the first segment and the second segment, and a cross-sectional diameter of the neck is smaller than at least one of a cross-sectional diameter of the first opening or a cross-sectional diameter of the second opening.

For the stent graft in some implementations, when the connecting stent is in the first configuration, the first segment is farther from the fenestration of the main stent than the second segment, the first opening is located at one end of the first segment away from the second segment, and the second opening is located at one end of the second segment away from the first segment. In the axial direction of the connecting stent, the cross-sectional diameter of the first segment gradually decreases from one end of the connecting stent where the first opening is located to the neck, and/or the cross-sectional diameter of the second segment gradually decreases from one end of the connecting stent where the second opening is located to the neck.

For the stent graft in some implementations, the connecting stent includes a connecting covering membrane and at least one support member. The connecting covering membrane forms the first segment and the second segment by the neck. The at least one support member is fixed to a peripheral wall of the first segment. In the axial direction of the connecting stent, a cross-sectional diameter of a support member gradually decreases from one end of the support member where the first opening is located to the neck.

For the stent graft in some implementations, the second segment is a flexible segment composed of the connecting covering membrane.

For the stent graft in some implementations, the connecting stent includes a connecting member. The connecting member at least partially surrounds and is fixed to the neck.

For the stent graft in some implementations, the connecting member is at least one of a suture set or a connecting rod. The suture set is at least one suture fixed by suturing to the neck.

For the stent graft in some implementations, the connecting stent in the second configuration is at least partially located in the lumen of the main stent.

For the stent graft in some implementations, when the connecting stent is in the second configuration, the second segment is at least partially located in the lumen of the main stent.

For the stent graft in some implementations, the first segment is at least partially overlappable with the second segment in the axial direction of the connecting stent. When the connecting stent is in the second configuration, the second segment and the first segment each are at least partially located in the lumen of the main stent.

For the stent graft in some implementations, when the connecting stent is in the second configuration, the first opening is located either outside the main stent or on the same plane as the cross section where the second opening is located.

For the stent graft in some implementations, a cross-sectional diameter of the first opening is larger than, smaller than, or equal to a cross-sectional diameter of the second opening.

For the stent graft in some implementations, the main stent further includes at least one radiopaque structure. Each of the at least one radiopaque structure at least partially surrounds and is fixed to the first opening of the connecting stent or the anchor stent, and/or each of the at least one radiopaque structure at least partially surrounds and is fixed to the second opening of the connecting stent or the anchor stent.

For the stent graft in some implementations, the stent graft includes at least one anchor stent. The at least one fenestration is implemented as at least two fenestrations defined on the sidewall of the main stent. The at least two fenestrations are a first fenestration and a second fenestration, respectively. The connecting stent and the at least one anchor stent are respectively fixed to the first fenestration and the second fenestration. The connecting stent is in communication with the lumen of the main stent through the first fenestration. The at least one anchor stent is in communication with the lumen of the main stent through the second fenestration. The at least one anchor stent is located at at least one of an inside of the lumen of the main stent or an outside of the lumen of the main stent.

For the stent graft in some implementations, the first fenestration is closer to a proximal end of the main stent than the second fenestration. The connecting stent is fixed to the first fenestration, and the at least one anchor stent is fixed to the second fenestration.

For the stent graft in some implementations, in the axial direction of the main stent from the proximal end of the main stent to a distal end of the main stent, the first fenestration and the second fenestration are spaced apart or not spaced apart from each other.

For the stent graft in some implementations, the at least one anchor stent is named at least one first stent. Each of the at least one first stent defines a first opening, a second opening, and an inner cavity extending between the first opening and the second opening. The first opening and the second opening are defined at two ends of a first stent in an axial direction of the first stent. The first stent is fixed to the second fenestration through one end of the first stent where the second opening is located, to enable the first stent to be in communication with the main stent. The first stent has a first configuration and a second configuration. The first stent is switchable between the first configuration and the second configuration. An axial length of the first stent in the first configuration is larger than an axial length of the first stent in the second configuration.

For the stent graft in some implementations, the first stent in the first configuration is located in the lumen of the main stent.

For the stent graft in some implementations, the first stent in the second configuration is partially located outside the main stent.

For the stent graft in some implementations, the first stent includes a first segment and a second segment fixed to the first segment. When the first stent is in the first configuration, the first segment of the first stent and the second segment of the first stent are arranged in the axial direction of the connecting stent, and a neck of the first stent is formed at a connection of the first segment and the second segment. The first opening of the first stent is located at one end of the first segment of the first stent away from the second segment. The second opening of the first stent is located at one end of the second segment of the first stent away from the first segment. A cross-sectional dimension of the first stent at the neck is smaller than at least one of a cross-sectional dimension of the first opening or a cross-sectional dimension of the second opening.

For the stent graft in some implementations, for each of the at least one first stent, in the axial direction of the first stent, a cross-sectional diameter of the first segment gradually decreases from one end of the first stent where the first opening is located to the neck, and/or a cross-sectional diameter of the second segment gradually decreases from one end of the first stent where the second opening is located to the neck.

For the stent graft in some implementations, a cross-sectional dimension of the first opening of the first stent is smaller than, larger than, or equal to a cross-sectional dimension of the second opening of the first stent.

For the stent graft in some implementations, the first stent includes a connecting covering membrane and at least one support member. The connecting covering membrane of the first stent forms the first segment and the second segment by the neck. The at least one support member is fixed to a peripheral wall of the first segment of the first stent.

For the stent graft in some implementations, at least a part of the second segment of the first stent is a flexible segment composed of the connecting covering membrane.

For the stent graft in some implementations, when the first stent is in the second configuration, the second segment is at least partially located outside the lumen of the main stent.

For the stent graft in some implementations, the first segment of the first stent is at least partially overlappable with the second segment of the first stent in the axial direction of the first stent. When the first stent is in the second configuration, the second segment and the first segment each are at least partially located outside the lumen of the main stent.

For the stent graft in some implementations, the axial length of the connecting stent in the first configuration is larger than the axial length of the first stent in the first configuration.

For the stent graft in some implementations, the stent graft includes at least two anchor stents. The at least one fenestration is implemented as at least three fenestrations defined on the sidewall of the main stent. The at least three fenestrations are a first fenestration, a second fenestration, and a third fenestration, respectively. The connecting stent and the at least two anchor stents are fixed to the first fenestration, the second fenestration, and the third fenestration, respectively. The connecting stent is in communication with the lumen of the main stent through the first fenestration. At least one of the at least two anchor stents is in communication with the lumen of the main stent through the second fenestration. At least one of the at least two anchor stents is in communication with the lumen of the main stent through the third fenestration. Any one of the at least two anchor stents is located in the lumen of the main stent or outside the lumen of the main stent.

For the stent graft in some implementations, the first fenestration is closer to a proximal end of the main stent than the second fenestration. The second fenestration is closer to the proximal end of the main stent than the third fenestration. The connecting stent is fixed to the first fenestration. At least one of the at least two anchor stents is fixed to the second fenestration. At least one of the at least two anchor stents is fixed to the third fenestration.

For the stent graft in some implementations, in the axial direction of the main stent from the proximal end of the main stent to a distal end of the main stent, the first fenestration, the second fenestration, and the third fenestration are spaced apart or not spaced apart from one another.

For the stent graft in some implementations, the stent graft includes at least one anchor stent. The connecting stent is implemented as at least two connecting stents. The at least one fenestration is implemented as at least three fenestrations defined on the sidewall of the main stent. The at least three fenestrations are a first fenestration, a second fenestration, and a third fenestration, respectively. The at least two connecting stents and at least one anchor stent are fixed to the first fenestration, the second fenestration, and the third fenestration, respectively. At least one of the at least two connecting stents is in communication with the lumen of the main stent through the first fenestration. At least one of the at least two connecting stents is in communication with the lumen of the main stent through the second fenestration. The at least one anchor stent is in communication with the lumen of the main stent through the third fenestration. At least one of the at least two connecting stents in the first configuration is located outside or in the lumen of the main stent.

For the stent graft in some implementations, the connecting stent is implemented as at least three connecting stents. The at least one fenestration is implemented as at least three fenestrations defined on the sidewall of the main stent. The at least three connecting stents are respectively fixed to the at least three fenestrations. The at least three connecting stents are respectively in communication with the lumen of the main stent through the at least three fenestrations fixed thereby. At least one of the at least three connecting stents in the first configuration is located outside or in the lumen of the main stent.

For the stent graft in some implementations, at least one of two ports of the at least one connecting stent in the axial direction of the connecting stent is configured as an oblique port.

For the stent graft in some implementations, at least one of two ports of each of the at least one anchor stent in an axial direction of an anchor stent is configured as an oblique port.

For the stent graft in some implementations, the at least two fenestrations are spaced apart or not spaced apart from each other in a direction from a proximal end of the main stent to a distal end of the main stent. Projections of the at least two fenestrations at least partially overlap in the axial direction of the main stent, and/or central axes of the at least two fenestrations coincide.

For the stent graft in some implementations, the at least three fenestrations are spaced apart or not spaced apart from one another in a direction from a proximal end of the main stent to a distal end of the main stent. Projections of the at least three fenestrations at least partially or completely overlap in the axial direction of the main stent, and/or central axes of the at least three fenestrations coincide.

For the stent graft in some implementations, in a direction from a proximal end of the main stent to a distal end of the main stent, lengths of the multiple fenestrations in the axial direction of the main stent sequentially increase.

For the stent graft in some implementations, in a direction from a proximal end of the main stent to a distal end of the main stent, lengths of the multiple fenestrations in a circumferential direction of the main stent sequentially increase.

For the stent graft in some implementations, the at least two fenestrations are defined on the sidewall of the main stent. The at least two fenestrations are the first fenestration and the second fenestration, respectively. The first fenestration is closer to a proximal end of the main stent than the second fenestration. A length of the second fenestration in a circumferential direction of the main stent is at least 50%-70% of a diameter of the main stent.

For the stent graft in some implementations, the at least two fenestrations are implemented as at least three fenestrations defined on the sidewall of the main stent. The at least three fenestrations are a first fenestration, a second fenestration, and a third fenestration, respectively. The first fenestration is closer to a proximal end of the main stent than the second fenestration. The second fenestration is closer to the proximal end of the main stent than the third fenestration. A length of the second fenestration in a circumferential direction of the main stent is at least 50%-70% of a diameter of the main stent, and/or a length of the third fenestration in the circumferential direction of the main stent is at least 60%-90% of the diameter of the main stent.

For the stent graft in some implementations, the main stent includes a main covering membrane and multiple main support frames fixed to the main covering membrane. The multiple main support frames are spaced apart in an axial direction of the main covering membrane. Each of the multiple main support frames includes multiple support rods sequentially connected at angles. Two adjacent angles in a circumferential direction of the main stent are respectively defined as a crest and a trough. The crest is closer to a proximal end of the main stent than the trough. For a main support frame of the multiple main support frames at at least one side of each of the at least one fenestration in the axial direction of the main stent, two adjacent support rods of the main support frame in the circumferential direction of the main stent define a surrounding angle therebetween. The surrounding angle and the two adjacent support rods defining the surrounding angle cooperatively form a semi-annular structure, and the semi-annular structure at least partially surrounds a fenestration.

For the stent graft in some implementations, the semi-annular structure is at least partially raised in a direction away from a central axis of the main stent.

For the stent graft in some implementations, the surrounding angle is raised in a direction away from a central axis of the main stent compared with other angles on the main support frame where the surrounding angle is located, or the surrounding angle and at least a part of each of the two support rods defining the surrounding angle are raised.

For the stent graft in some implementations, the main stent includes a first support segment, a recessed segment, and a second support segment extending sequentially from a proximal end of the main stent to a distal end of the main stent. A radial dimension of the first support segment is larger than each of a radial dimension of the recessed segment and a radial dimension of the second support segment, and the radial dimension of the recessed segment is smaller than the radial dimension of the second support segment, to enable the recessed segment to be at least partially recessed in a radial direction of the main stent.

For the stent graft in some implementations, at least two fenestrations are located on the recessed segment; or the at least one fenestration is located on the first support segment.

For the stent graft in some implementations, the recessed segment includes an avoidance segment and at least one flared portion. The at least one flared portion extends from a proximal end of the avoidance segment towards the proximal end of the main stent. The recessed segment is fixed to a distal end of the first support segment through a proximal end of the flared portion. In a circumferential direction of each of the at least one flared portion, each of the at least one flared portion is at least partially radially enlarged in a direction away from a central axis of the recessed segment.

For the stent graft in some implementations, one of the at least one fenestration close to the proximal end of the main stent is located on one of the at least one flared portion close to the proximal end of the main stent; or each of the at least one fenestration is located on the avoidance segment.

A stent system is provided in some implementations. The stent system includes a branch stent and the stent graft described above. The branch stent is insertable into the connecting stent through either of the first opening of the connecting stent and the second opening of the connecting stent; and/or the branch stent is insertable into an anchor stent of the stent graft.

A stent system is provided in some implementations. The stent system includes the stent graft described above and an interventional instrument for delivering the stent graft to a target position. The interventional instrument includes a guide head, an interventional sheath core fixed at a distal end of the guide head, an interventional sheath tube sleeved over the interventional sheath core, and an operation handle for controlling axial movement of the interventional sheath tube. A gap is defined between the interventional sheath tube and the interventional sheath core for accommodating the stent graft that is radially compressed. A proximal end of the stent graft is detachably fixed to the interventional sheath core.

For the stent system in some implementations, a pre-embedded guidewire is routed through the connecting stent. The pre-embedded guidewire is also routed through the main stent. One end of the pre-embedded guidewire is located outside the connecting stent, and another end of the pre-embedded guidewire is located outside the interventional sheath tube.

An application method for a stent graft is provided in some implementations. The stent graft includes the stent graft described above. The application method includes the following. When the stent graft is implanted, the first fenestration or the second fenestration is aligned with a target branch vessel first and then other fenestrations are aligned with the target branch vessel.

The following embodiments of the present disclosure will be described with reference to the accompanying drawings of the embodiments of the present disclosure. It is apparent that the embodiments described are only a part of the embodiments of the present disclosure, rather than all of the embodiments. Based on the embodiments of the present disclosure, all other embodiments obtained by those of ordinary skill in the art without creative effort shall fall within the scope of protection of the present disclosure.

In the description of the embodiments of the present disclosure, it should be noted that the term "integrally formed" refers to that, in the process of forming one of multiple components, the component is connected to other components, without the need to connect two components together by further processing (such as bonding, welding, snap-fit connection, or screw connection). The terminology used in the specification and the claims of the present disclosure and the accompanying drawings is merely for the purpose of describing particular embodiments, and is not intended to limit the present disclosure. The terms "first," "second," and the like used in the specification and the claims of the present disclosure and the accompanying drawings are only for distinguishing different objects, rather than for describing a specific sequence. The directional terms mentioned in the embodiments of the present disclosure, such as "inner", "outer", "side", and the like, are merely with reference to the directions shown in the accompanying drawings. Therefore, the directional terms used are for better and clearer description and understanding of the embodiments of the present disclosure, and are not intended to indicate or imply that the referred device or element must have a specific orientation, be constructed in a specific orientation, or operated in a specific orientation, and thus should not be construed as a limitation to the embodiments of the present disclosure.

In endovascular interventional therapy, a proximal end of an endovascular stent refers to one end of the endovascular stent that is close to the human heart when the endovascular stent is used for interventional therapy, and a distal end of the vascular stent refers to one end of the vascular stent that is away from the human heart when the vascular stent is used for interventional therapy.

In the branched stent technology, one or more branches are embedded in or extended from the main stent, and during the operation, bridging stents are implanted into target vessels through respective branches to reconstruct the visceral arteries. However, the embedded branches increase the lumen loss rate of the main stent, and the extended branches require that there be sufficient space within the vessel for the branches to expand. In surgery, difficulties exist regardless of whether the extended branches or the embedded branches are selected.

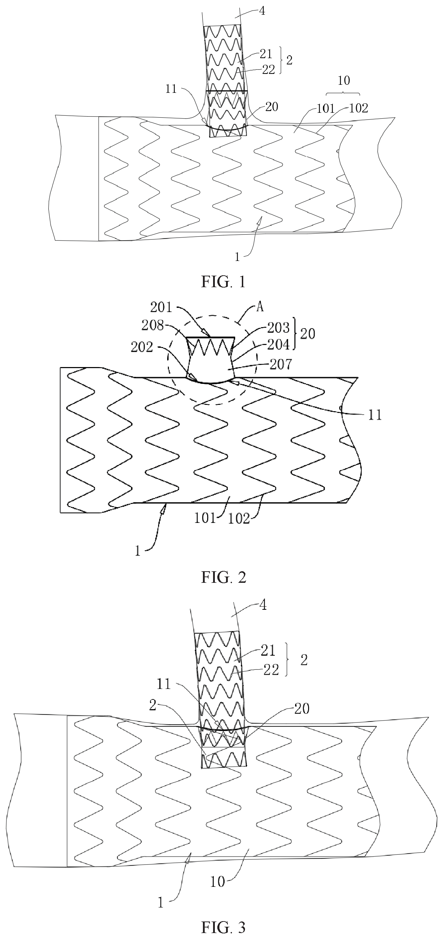

Reference can be made to FIG. 1 and FIG. 2, where FIG. 1 is a schematic structural view of a connecting stent 20 in an extended state on a stent system provided in a first embodiment of the present disclosure, and FIG. 2 is a schematic structural view of the connecting stent 20 in the extended state on a stent graft illustrated in FIG. 1. The stent system includes a stent graft 1 and a branch stent 2. The stent graft 1 includes a main stent 10 for reconstructing the trunk vessel and a connecting stent 20 for anchoring the branch stent 2. The branch stent 2 is inserted into the connecting stent 20 for reconstructing a branch vessel 4.

The main stent 10 defines two openings at two ends of the main stent 10 in an axial direction of the main stent 10 and a lumen extending axially between the two openings. The main stent 10 defines a fenestration 11 on a sidewall of the main stent 10. The fenestration 11 is in communication with the lumen of the main stent 10. Either end of the connecting stent 20 is fixed to the sidewall of the main stent 10, and the connecting stent 20 is in communication with the lumen of the main stent 10 through the fenestration 11 of the main stent 10. Exemplarily, the main stent 10 includes a main covering membrane 101 and a main support frame 102 fixed to the main covering membrane 101.

In some implementations, at least one fenestration 11 is defined on the sidewall of the main stent 10.

In some implementations, the fenestration 11 is defined on the main covering membrane 101.

In some implementations, the main stent 10 is at least partially recessed at the middle portion of the main stent 10. In other words, a peripheral wall of the main stent 10 at least close to the connecting stent 20 may be recessed towards the lumen of the main stent 10, which is conducive to reducing the extrusion of the branch stent 2 by the main stent 10 after completion of the interventional treatment. It can be understood that the outer peripheral wall of the main stent 10 at the middle portion of the main stent 10 may all be recessed, so that the main stent 10 has a shape of being large at openings of the two ends of the main stent 10 and being small at the middle portion of the main stent 10.

The connecting stent 20 defines a first opening 201, a second opening 202, and an inner cavity extending between the first opening 201 and the second opening 202. The first opening 201 and the second opening 202 are defined at two ends of the connecting stent 20 in an axial direction of the connecting stent 20. Either of the first opening 201 and the second opening 202 is fixed to the fenestration 11 of the main stent 10, so that the inner cavity of the connecting stent 20 is in communication with the lumen of the main stent 10 through the fenestration 11.

In some implementations, either of the first opening 201 and the second opening 202 is fixed to at least one fenestration 11 of the main stent 10, so that the inner cavity of the connecting stent 20 is in communication with the lumen of the main stent 10 through the fenestration 11.

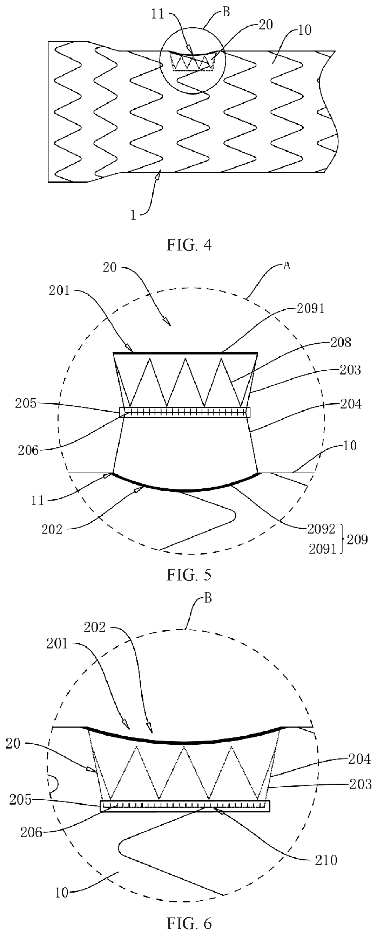

Reference can be made to FIG. 3 and FIG. 4, where FIG. 3 is a schematic structural view of a connecting stent 20 in a retracted state on the stent system illustrated in FIG. 1, and FIG. 4 is a schematic structural view of the connecting stent 20 in the retracted state on a stent graft 1 illustrated in FIG. 3. The connecting stent 20 has a first configuration (e.g., the connecting stent 20 is in an extended state) and a second configuration (e.g., the connecting stent 20 is in a retracted state). An axial length of the connecting stent 20 in the first configuration is larger than an axial length of the connecting stent 20 in the second configuration. The connecting stent 20 in the first configuration can form an extended branch, that is, the connecting stent 20 in the first configuration is located on the outer sidewall of the main stent 10. The connecting stent 20 in the second configuration can form an embedded branch, that is, the connecting stent 20 in the second configuration is at least partially located within the lumen of the main stent 10. The branch stent 2 is inserted into the connecting stent 20, that is, the branch stent 2 is insert-fitted with the connecting stent 20. For example, the branch stent 2 is partially inserted into the connecting stent 20 along its own axial length, and can be used to reconstruct various types of branch vessels 4 to meet different needs during surgery.

As illustrated in FIG. 2, when the connecting stent 20 is in the first configuration, the connecting stent 20 can extend outward beyond the main stent 10 to form an extended branch. In this way, the connecting stent 20 can be located outside the main stent 10, thereby reducing the lumen loss rate of the main stent 10.

As illustrated in FIG. 4, when the connecting stent 20 is in the second configuration, the connecting stent 20 can be retracted towards the lumen of the main stent 10 through the fenestration 11 of the main stent 10, to form an embedded branch. In this way, the connecting stent 20 can be at least partially located in the lumen of the main stent 10. There is no need to have a certain space in the trunk vessel for the connecting stent 20 to extend. It is suitable for reconstructing some vessels in which there is no sufficient space in the trunk vessel to be compatible with the extended branch structure, such as some vessels with complex morphological structures.

In some other implementations, the connecting stent 20 in the first configuration is located within the lumen of the main stent 10.

In some other implementations, the connecting stent 20 in the second configuration is located outside the main stent 10.

As illustrated in FIG. 2, when the connecting stent 20 is in the first configuration, one end of the connecting stent 20 where the second opening 202 is located is fixed to the fenestration 11 of the main stent 10. In some implementations, a cross-sectional diameter of the first opening 201 is larger than a cross-sectional diameter of the second opening 202.

In other implementations, the cross-sectional diameter of the first opening 201 may be smaller than the cross-sectional diameter of the second opening 202.

The first opening 201 is located at a distal end of the connecting stent 20. The second opening 202 is located at a proximal end of the connecting stent 20. The proximal end of the connecting stent 20 is fixed to the main stent 10, and the inner cavity of the connecting stent 20 is in communication with the lumen of the main stent 10. For example, the inner cavity of the connecting stent 20 is in communication with the lumen of the main stent 10 through the at least one fenestration 11.

In some implementations, the second opening 202 of the connecting stent 20 is sutured to the fenestration 11 of the main stent 10 by a suture. The second opening 202 of the connecting stent 20 is in communication with the fenestration 11 of the main stent 10.

In some implementations, the second opening 202 of the connecting stent 20 is sutured to the at least one fenestration 11 of the main stent 10 by a suture. The second opening 202 of the connecting stent 20 is in communication with the main stent 10 through the at least one fenestration 11.

Reference can be made to FIG. 5, which is a partial enlarged view of the stent graft 1 illustrated in FIG. 2 at Circle A. In some implementations, the connecting stent 20 includes a first segment 203 and a second segment 204 fixed to the first segment 203.

In some implementations, the first opening 201 of the connecting stent 20 is located at one end of the first segment 203 away from the second segment 204. The second opening 202 of the connecting stent 20 is located at one end of the second segment 204 away from the first segment 203.

A neck 205 of the connecting stent 20 is formed at a connection of the first segment 203 and the second segment 204. That is, the connecting stent 20 forms the first segment 203 and the second segment 204 by the neck 205. When the connecting stent 20 is in the first configuration, the first segment 203 and the second segment 204 are arranged in the axial direction of the connecting stent 20 and are in communication with each other. When the connecting stent 20 is in the first configuration, the first segment 203 of the connecting stent 20 is expanded relative to the second segment 204. At this time, the axial length of the connecting stent 20 is the sum of the length of the first segment 203 and the length of the second segment 204. The connecting stent 20 in the first configuration is located outside the main stent 10, the first segment 203 is farther from the fenestration 11 of the main stent 10 than the second segment 204, and the connecting stent 20 is an extended branch, thereby reducing the lumen loss rate of the main stent 10.

In some implementations, a cross-sectional dimension of the connecting stent 20 at the neck 205 is smaller than at least one of a cross-sectional dimension of the first opening 201 or a cross-sectional dimension of the second opening 202 of the connecting stent 201.

In some implementations, the connecting stent 20 includes a first segment 203 and a second segment 204 fixed to the first segment 203. A cross-sectional dimension of one end of the first segment 203 close to the second segment 204 is smaller than a cross-sectional dimension of one end of the first segment 203 away from the second segment 204, that is, the cross-sectional dimension of the end of the first segment 203 close to the second segment 204 is smaller than the cross-sectional dimension of the first opening 201 of the connecting stent 20, so that the connection of the first segment 203 and the second segment 204 forms the neck 205 of the connecting stent 20. Alternatively, the cross-sectional dimension of the end of the second segment 204 close to the first segment 203 is smaller than the cross-sectional dimension of the end of the second segment 204 away from the first segment 203, that is, the cross-sectional dimension of the end of the second segment 204 close to the first segment 203 is smaller than the cross-sectional dimension of the second opening 202 of the connecting stent 20, so that the neck 205 of the connecting stent 20 is formed at the connection of the first segment 203 and the second segment 204.

In some implementations, the connecting stent 20 includes a first segment 203 and a second segment 204 fixed to the first segment 203. A cross-sectional dimension of one end of the first segment 203 close to the second segment 204 is smaller than a cross-sectional dimension of one end of the first segment 203 away from the second segment 204, and a cross-sectional dimension of one end of the second segment 204 close to the first segment 203 is smaller than a cross-sectional dimension of one end of the second segment 204 away from the first segment 203, so that the neck 205 of the connecting stent 20 is formed at the connection of the first segment 203 and the second segment 204.

In some implementations, a cross-sectional diameter of the neck 205 is smaller than each of the cross-sectional diameter of the first opening 201 and the cross-sectional diameter of the second opening 202.

Exemplarily, the first opening 201 is located at one end of the first segment 203 away from the second segment 204. In the axial direction of the connecting stent 20, a cross-sectional diameter of the first segment 203 gradually decreases from one end of the connecting stent 20 where the first opening 201 is located to the neck 205.

Exemplarily, the second opening 202 is located at one end of the second segment 204 away from the first segment 203. In the axial direction of the connecting stent 20, a cross-sectional diameter of the second segment 204 gradually decreases from one end of the connecting stent 20 where the second opening 202 is located to the neck 205.

In some implementations, both the first segment 203 and the second segment 204 are designed to have a tapered shape, which facilitates a closer fitting between the first segment 203 and the second segment 204, so that the branch stent 2 can be more stably inserted into the connecting stent 20, or that the branch stent 2 can be more stably inserted into the connecting stent 20 in the second configuration.

In some implementations, both the first segment 203 and the second segment 204 of the connecting stent 20 are in the shape of a truncated cone. The connecting stent 20 is in the shape of a slender waist, an hourglass, or the like. Compared with a cylindrical structure, the design of the connecting stent 20 in the present disclosure (for example, the connecting stent 20 is in the shape of a slender waist or an hourglass) allows the connecting stent 20 to bend at the neck 205, so that the connecting stent 20 has expandability, thereby having an extended configuration and a retracted configuration.

In some implementations, either of the first segment 203 of the connecting stent 20 and the second segment 204 of the connecting stent 20 is in the shape of a truncated cone, and either of the first segment 203 of the connecting stent 20 and the second segment 204 of the connecting stent 20 is in the shape of a taper.

In some implementations, a ratio of the cross-sectional diameter of the first opening 201 or the second opening 202 to the cross-sectional diameter of the neck 205 is greater than 1. Exemplarily, the ratio of the cross-sectional diameter of the first opening 201 or the second opening 202 to the cross-sectional diameter of the neck 205 ranges from 1.1 to 2.1.

Exemplarily, the ratio of the cross-sectional diameter of the first opening 201 or the second opening 202 to the cross-sectional diameter of the neck 205 is 1.2. At this time, the connecting stent 20 has good expandability.

In some other implementations, the connecting stent 20 at least partially consists of a corrugated tube in the axial direction of the connecting stent 20, thereby adjusting a variety of different axial extension and retraction lengths.

Reference can be made to FIG. 6, which is a partial enlarged view of the stent graft 1 illustrated in FIG. 4 at Circle B. The first segment 203 and the second segment 204 of the connecting stent 20 can overlap in the axial direction of the connecting stent 20, that is, the connecting stent 20 is in the second configuration. When the connecting stent 20 is in the second configuration, the first segment 203 and the second segment 204 can overlap in the axial direction of the connecting stent 20.

The axial length of the connecting stent 20 is smaller than the sum of the length of the first segment 203 and the length of the second segment 204, that is, the axial length of the connecting stent 20 is smaller than the axial length of the connecting stent 20 in the first configuration, so that the space occupied by the connecting stent 20 is reduced.

In some implementations, when the connecting stent 20 bends at the neck 205 (e.g., when the connecting stent 20 is converted to the second configuration), the connecting stent 20 defines a third opening 210 at the neck 205. The third opening 210 is located in the lumen of the main stent 10. The first opening 201 and the second opening 202 are located on the same side of the third opening 210. For example, the first opening 201 is farther from the third opening 210 relative to the second opening 202; or the first opening 201 is located on the same plane as the cross section where the second opening 202 is located; or the first opening 201 is closer to the third opening 210 relative to the second opening 202. When the connecting stent 20 is in the second configuration, the first segment 203 and the second segment 204 of the connecting stent 20 each are at least partially located in the lumen of the main stent 10, and the connecting stent 20 is an embedded branch, which is suitable for reconstructing some vessels in which there is no sufficient space near a connection of the trunk vessel and the branch vessel 4 to be compatible with the extended branch structure. In addition, when the connecting stent 20 is retracted into the lumen of the main stent 10, the axial length of the connecting stent 20 is reduced, and the space occupied by the connecting stent 20 in the lumen of the main stent 10 is relatively small, thereby reducing the lumen loss rate of the main stent 10 to a certain extent.

In some implementations, the first opening 201 is located outside the main stent 10. At this time, the second segment 204 of the connecting stent 20 is located in the lumen of the main stent 10. A part of the first segment 203 of the connecting stent 20 is partially located in the lumen of the main stent 10, and the other part of the first segment 203 of the connecting stent 20 is located outside the main stent 10. Since the cross-sectional diameter of the first opening 201 of the connecting stent 20 is larger than or smaller than the cross-sectional diameter of the second opening 202 of the connecting stent 20, a part of the peripheral wall of the first segment 203 of the connecting stent 20 is snap-fitted with the fenestration 11 of the main stent 10, thereby enhancing the connection firmness between the connecting stent 20 and the main stent 10.

In another implementation, the first opening 201 of the connecting stent 20 is located on the same plane as the cross section where the second opening 202 is located. In this way, most of the first segment 203 and the second segment 204 of the connecting stent 20 are located in the lumen of the main stent 10. Therefore, the space occupied by the connecting stent 20 at the branch vessel 4 is reduced, thereby facilitating improvement of the space utilization rate of the inner cavity of the branch vessel 4.

As illustrated in FIG. 5 and FIG. 6, the connecting stent 20 is switchable between the first configuration and the second configuration, to form an extended branch or an embedded branch. The connecting stent 20 is switched from the first configuration to the second configuration, so as to reconstruct some vessels in which there is no sufficient space near a connection of the trunk vessel and the branch vessel 4 to be compatible with the extended branch structure. When the connecting stent 20 is in the first configuration, by pushing the first segment 203 towards the lumen of the main stent 10 in the axial direction of the connecting stent 20, the connecting stent 20 bends at the neck 205, and the first segment 203 at least partially overlaps the second segment 204 in the axial direction of the connecting stent 20, with the first segment 203 being at least partially pushed into the lumen of the main stent 10. In this way, the connecting stent 20 is switched from the first configuration to the second configuration, the connecting stent 20 is changed from the extended branch to the embedded branch, and the branch stent 2 is inserted into the connecting stent 20 to reconstruct the branch vessel 4.

In some other implementations, it is also possible to pull the first segment 203 from a convenient location (e.g., from the lumen of the main stent 10) to move into the lumen of the main stent 10, so that the first segment 203 is at least partially located within the lumen of the main stent 10.

When the trunk vessel to be reconstructed has sufficient space at the branch vessel 4 to be compatible with the extended branch structure, the connecting stent 20 is switched from the second configuration to the first configuration to reduce the lumen loss rate of the main stent 10.

In some implementations, when the connecting stent 20 is in the second configuration, for example, a balloon can be inserted into the inner cavity of the connecting stent 20, and the balloon can be inflated and fit against at least a part of the inner peripheral wall of the connecting stent 20. The inflated balloon engages with the inner peripheral wall of the connecting stent 20, and the connecting stent 20 is stretched and pulled out of the lumen of the main stent 10 by using the friction between the balloon and the inner peripheral wall of the connecting stent 20. Therefore, the connecting stent 20 is stretched from the embedded branch to the extended branch, the connecting stent 20 is switched from the second configuration to the first configuration, and the branch stent 2 is inserted into the connecting stent 20 to reconstruct the branch vessel 4, thereby reducing the lumen loss rate of the main stent 10.

It can be understood that by controlling the extent to which the connecting stent 20 is pulled out of the lumen of the main stent 10, the degree of overlap between the first segment 203 and the second segment 204 in the axial direction of the connecting stent 20 can vary, so as to adapt the angulation between the trunk vessel and the branch vessel 4 and the space between the trunk vessel and the branch vessel 4 of different patients.

In other implementations, other methods can also be used, for example, by directly pulling the connecting stent 20 to form the extended branch, and then inserting the branch stent 2 to reconstruct the branch vessel 4. Specifically, the present disclosure does not limit this.

As illustrated in FIG. 5 and FIG. 6, in some implementations, the connecting stent 20 further includes a connecting member 206. The connecting member 206 at least partially surrounds and is fixed to the neck 205. The connecting member 206 is configured to connect the first segment 203 and the second segment 204. Exemplarily, the connecting member 206 is a suture set. The suture set is at least one suture fixed by suturing to the neck 205.

In some implementations, the first segment 203 and the second segment 204 of the connecting stent 20 are sutured to the neck 205 by a suture set.

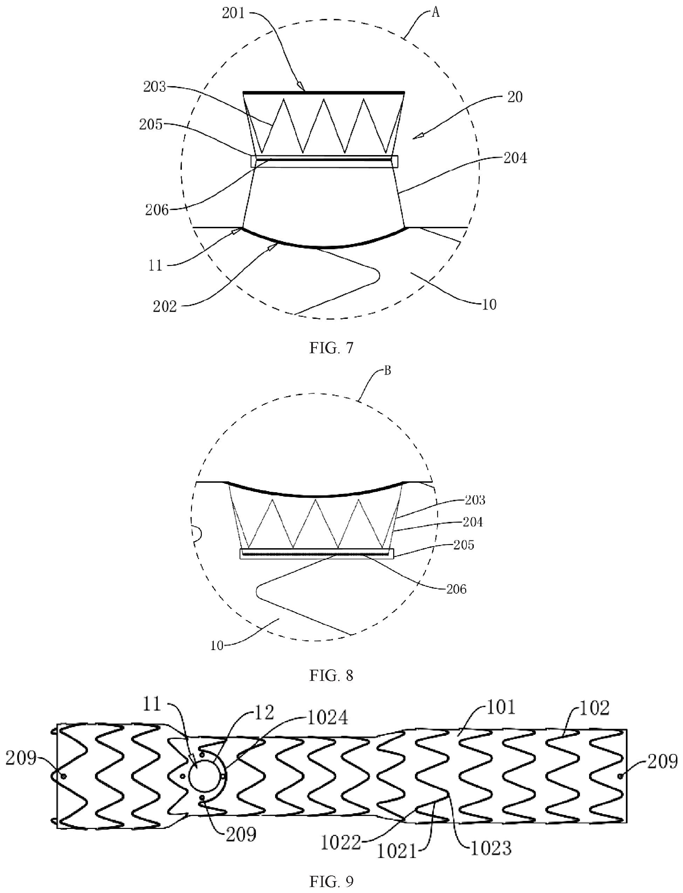

Reference can be made to FIG. 7 and FIG. 8, where FIG. 7 is a schematic structural view of the stent graft 1 illustrated in FIG. 5 in other implementations, and FIG. 8 is a schematic structural view of the stent graft 1 illustrated in FIG. 6 in other implementations. In some implementations, the connecting member 206 is an annular connecting rod. The first segment 203 and the second segment 204 of the connecting stent 20 are fixed to the neck 205 by the connecting rod. The connecting rod at least partially surrounds and is fixed to the neck 205.

In some implementations, the connecting member 206 is provided at the neck 205 of the connecting stent 20. In this way, the first segment 203 and the second segment 204 of the connecting stent 20 can be machined separately. For example, the first segment 203 and the second segment 204 are first machined into truncated cone shapes, respectively, and then the first segment 203 and the second segment 204 are connected by the connecting member 206 to form the slender-shaped or hourglass-shaped connecting stent 20.

The connecting member 206 serves to positionally divide the connecting stent 20 into the first segment 203 and the second segment 204, that is, to facilitate defining the respective positions of the first segment 203 and the second segment 204, thereby facilitating the transition of the connecting stent 20 between the first configuration and the second configuration.

In other implementations, the connecting stent 20 is an integrally formed structural member. That is, the first segment 203, the neck 205, and the second segment 204 of the connecting stent 20 are integrally formed. In this way, the connection between the first segment 203 and the neck 205, the connection between the neck 205 and the second segment 204, and the connection between the first segment 203 and the second segment 204, can be established without other components, so that the connecting stent 20 has a simple structure with relatively good overall structural sealing.

As illustrated in FIG. 5, in one or more embodiments, the connecting stent 20 includes a connecting covering membrane 207. The configuration of the connecting covering membrane 207 changes with the configuration of the connecting stent 20, for example, each of the connecting covering membrane 207 and the connecting stent 20 has a neck 205. The connecting covering membrane 207 forms a first segment 203 and a second segment 204 by the neck 205. The connecting covering membrane 207 forms a tubular body structure to which the stent 20 is connected. That is, the connecting covering membrane 207 encloses to define the inner cavity of the connecting stent 20. In some implementations, a material of the connecting covering membrane 207 is similar to a material of the main covering membrane 101, which is not repeated herein. It can be understood that the connecting covering membrane 207 is a flexible material, which facilitates folding and unfolding of the connecting covering membrane 207.

As illustrated in FIG. 5, in some implementations, the connecting stent 20 further includes at least one support member 208. The at least one support member 208 is fixed to the peripheral wall of the first segment 203. Exemplarily, the support member 208 is disposed in the circumferential direction of the connecting stent 20. The support member 208 is sutured and fixed to the peripheral wall of the first segment 203 by a suture.

The configuration of the support member 208 conforms to the configuration of the first segment 203. Exemplarily, in the axial direction of the connecting stent 20, a cross-sectional diameter of the support member 208 gradually decreases from one end of the connecting stent 20 where the first opening 201 is located.

Exemplarily, the structure of the support member 208 may be one or more of a sawtooth shape, a wavy shape, or a straight shape.

In some implementations, the structure of the support member 208 is the same as the structure of any main support frame 102.

Exemplarily, the number of support members 208 may be one or more. When there are multiple support members 208, the multiple support members 208 are arranged in the axial direction of the connecting stent 20.

Exemplarily, a material of the support member 208 is similar to a material of the main support frame 102. Specifically, the details will not be described here.

In one or more embodiments, the first segment 203 of the connecting stent 20 is fixed with a support member 208. The support member 208 supports the first segment 203, so that the first segment 203 of the connecting stent 20 has a certain strength, such as a radial supporting force. At this time, the first segment 203 of the connecting stent 20 may serve as a support segment. The support member 208 increases the anchoring force between the connecting stent 20 and the branch stent 2, thereby improving the connection firmness between the connecting stent 20 and the branch stent 2.

In some implementations, the second segment 204 of the connecting stent 20 is a flexible segment formed by the connecting covering membrane 207. The second segment 204 is folded towards the lumen of the main stent 10, so that the connecting stent 20 can be at least partially located in the lumen of the main stent 10.

In other implementations, the second segment 204 of the connecting stent 20 is also fixed with a support member 208.

In other implementations, the second segment 204 of the connecting stent 20 is also fixed with the support member 208, and the stiffness of the support member 208 on the second segment 204 is less than the stiffness of the support member 208 on the first segment 201, or the wire diameter of the support member 208 on the second segment 204 is smaller than the wire diameter of the support member 208 on the first segment 201, so that the strength of the second segment 204 of the connecting stent 20 is less than the strength of the first segment 203 of the connecting stent 20.

In some other implementations, at least one of the first segment 203 of the connecting stent 20 or the second segment 204 of the connecting stent 20 consists of a corrugated tube in the axial direction of the connecting stent 20, thereby adjusting a variety of different axial extension and retraction lengths.

In some implementations, the second segment 204 of the connecting stent 20 consists of a corrugated tube in the axial direction of the connecting stent 20, thereby adjusting a variety of different axial extension and retraction lengths.

As illustrated in FIG. 5, in some implementations, the main stent 10 further includes at least one radiopaque structure 209. The radiopaque structure 209 at least partially surrounds and is fixed to the first opening 201, or the radiopaque structure 209 at least partially surrounds and is fixed to the second opening 202.

Exemplarily, the connecting stent 20 includes a first annular radiopaque member 2091. The first annular radiopaque member 2091 surrounds the first opening 201 and is fixed to the connecting covering membrane 207, thereby reinforcing a preset shape of the first opening 201 of the connecting stent 20. Specifically, the first annular radiopaque member 2091 may be fixed around one end of the connecting stent 20 where the first opening 201 is located; alternatively, the first annular radiopaque member 2091 may be fixed to an end surface of the connecting stent 20 where the first opening 201 is located.

In one or more embodiments, the connecting stent 20 further includes a second annular radiopaque member 2092. The second annular radiopaque member 2092 surrounds the second opening 202 and is fixed to the connecting covering membrane 207, thereby reinforcing a preset shape of the second opening 202 of the connecting stent 20. Specifically, the second annular radiopaque member 2092 may be fixed around one end of the connecting stent 20 where the second opening 202 is located; alternatively, the second annular radiopaque member 2092 may be fixed to an end surface of the connecting stent 20 where the second opening 202 is located.

In some implementations, the first annular radiopaque member 2091 and the second annular radiopaque member 2092 are provided on the connecting stent 20. The first annular radiopaque member 2091 surrounds the first opening 201. The second annular radiopaque member 2092 surrounds the second opening 202. When it is necessary to insert the branch stent 2 into the connecting stent 20, the position of the first annular radiopaque member 2091 and the position of the second annular radiopaque member 2092 can be clearly observed by an imaging device, that is, the position of the first opening 201 and the position of the second opening 202 of the connecting stent 20 can be clearly observed, thereby facilitating the assembly of the connecting stent 20 and the branch stent 2.

Reference can be made to FIG. 9, which is a schematic structural view of a main stent 10 in some implementations. In some implementations, there are multiple main support frames 102, and the multiple main support frames 102 are arranged in the axial direction of the main stent 10.

Each of the multiple main support frames 102 includes multiple support rods 1021 sequentially connected at angles, two adjacent angles in the circumferential direction of the main stent 10 are respectively defined as a crest 1022 and a trough 1023, and the crest 1022 is closer to the proximal end of the main stent 10 than the trough 1023.

In some implementations, for a main support frame 11 of the multiple main support frames 102 at at least one side of each of the at least one fenestration 11 in the axial direction of the main stent 10, two adjacent support rods 1021 of the main support frame 102 in the circumferential direction of the main stent 10 define a surrounding angle 1024 therebetween. The surrounding angle 1024 is configured as a rounded angle. The surrounding angle 1024 and the two adjacent support rods 1021 defining the surrounding angle 1024 cooperatively form a semi-annular structure, and the semi-annular structure at least partially surrounds a fenestration 11.

In some implementations, the fenestration 11 is fixed with a support ring 12 for reinforcing the configuration of the fenestration 11.

In some implementations, two ends of the support ring 12 in the axial direction of the main stent 10 are raised, that is, the two ends of the support ring 12 in the axial direction of the main stent 10 are raised compared with other regions of the support ring 12, which is conducive to lifting the main covering membrane 101 in a direction away from a central axis of the main stent 10. Therefore, the lumen loss rate of the main stent 10 can be further reduced, more blood can flow through the lumen of the main stent 10, and more blood can flow to the branch vessel 4 through the fenestration 11, thereby reducing the occurrence of complications.

Referring to FIG. 1 and FIG. 2, the branch stent 2 includes a branch covering membrane 21 and a branch support frame 22 fixed to the branch covering membrane 21. There are multiple branch support frames 22. Exemplarily, the branch support frame 22 is fixed to a peripheral wall, for example, an outer peripheral wall, of the branch covering membrane 21. The structure, shape, and material of the branch covering membrane 21 are similar to the structure, shape, and material of the main covering membrane 101, and the structure, shape, and material of the branch support frame 22 are similar to the structure, shape, and material of the main support frame 102, which are not repeated herein.

The first opening 201 or the second opening 202 of the connecting stent 20 is fixed to the main stent 10. The branch stent 2 can be inserted into the connecting stent 20 through the first opening 201 or the second opening 202 of the connecting stent 20. The inner cavity of the branch stent 2 is in communication with the lumen of the main stent 10.

In some implementations, the second opening 202 of the connecting stent 20 is fixed to the main stent 10. The branch stent 2 can be inserted into the connecting stent 20 through the first opening 201 of the connecting stent 20. The inner cavity of the branch stent 2 is in communication with the lumen of the main stent 10.

In some implementations, an outer diameter of the branch stent 2 is larger than the cross-sectional diameter of the neck 205 of the connecting stent 20. The branch stent 2 is in interference fit with the connecting stent 20, so that a part of the outer peripheral wall of the branch stent 2 can fit against a part of the inner peripheral wall of the connecting stent 20, thereby enhancing the connection firmness between the branch stent 2 and the connecting stent 20, which is conducive to effectively preventing endoleak.

In some implementations, the outer diameter of the branch stent 2 is greater than the cross-sectional diameter at any point of the connecting stent 20.

In some implementations, by providing the branch support frame 22 on the branch covering membrane 21, the branch support frame 22 can increase the friction between the outer peripheral wall of the branch stent 2 and the inner peripheral wall of the connecting stent 20, thereby improving the connection strength of the branch stent 2 and the connecting stent 20, and making the connection between the branch stent 2 and the connecting stent 20 more firm. In addition, relative movement between the connecting stent 20 and the branch stent 2 is unlikely to occur, and the branch stent 2 is unlikely to fall off from the connecting stent 20.

In some implementations, when the connecting stent 20 is in the first configuration, a proximal end of the branch stent 2 passes through an opening (e.g., the first opening 201 as illustrated in FIG. 1 and FIG. 2) of the connecting stent 20 away from the fenestration 11, passes through the inner cavity of the connecting stent 2, and extends out of an opening (e.g., the second opening 202 as illustrated in FIG. 1 and FIG. 2) of the connecting stent 20 close to the fenestration 11. The proximal end of the branch stent 2 is located outside the connecting stent 20, that is, the proximal end of the branch stent 2 is located in the lumen of the main stent 10. As illustrated in FIG. 1 and FIG. 2, when the connecting stent 20 is in the first configuration, the proximal end of the branch stent 2 passes through the first opening 201 of the connecting stent 20 and the inner cavity of the connecting stent 20 and extends out of the second opening 202, and the proximal end of the branch stent 2 is located in the lumen of the main stent 10.

In other implementations, the proximal end of the branch stent 2 does not extend out of the second opening 202. That is, the proximal end of the branch stent 2 is located in the inner cavity of the connecting stent 20, and the proximal end of the branch stent 2 does not extend into the lumen of the main stent 10. The branch stent 2 does not extend into the lumen of the main stent 10, so that the lumen loss rate of the main stent 10 can be reduced.

When the connecting stent 20 is in the second configuration, the proximal end of the branch stent 2 passes through the first opening 201 and the second opening 202 of the connecting stent 20 and extends out of the third opening 210, and the proximal end of the branch stent 2 is located in the lumen of the main stent 10.

In other implementations, the proximal end of the branch stent 2 does not extend out of the third opening 210. That is, the proximal end of the branch stent 2 is located in the inner cavity of the connecting stent 20 in the second configuration, and the proximal end of the branch stent 2 does not extend into the lumen of the main stent 10.

On condition that there is insufficient space at the trunk vessel to be compatible with the extended branch structure, the connecting stent 20 is switched to the second configuration to form an embedded branch, thereby reducing the space occupied by the connecting stent 20 in the vessel, and the branch stent 2 is inserted into the connecting stent 20 to reconstruct the branch vessel 4.

For a vessel in which there is sufficient space at the trunk vessel to be compatible with the extended branch structure, the connecting stent 20 is switched to the first configuration to form an extended branch, and the branch stent 2 is inserted into the connecting stent 20 to reconstruct the branch vessel 4, thereby reducing the lumen loss rate of the main stent 10.

In other implementations, the first opening 201 of the connecting stent 20 is fixed to the main stent 10. The branch stent 2 can be inserted into the connecting stent 20 through the second opening 202 of the connecting stent 20. The inner cavity of the branch stent 2 can be in communication with the lumen of the main stent 10 through the first opening 201.

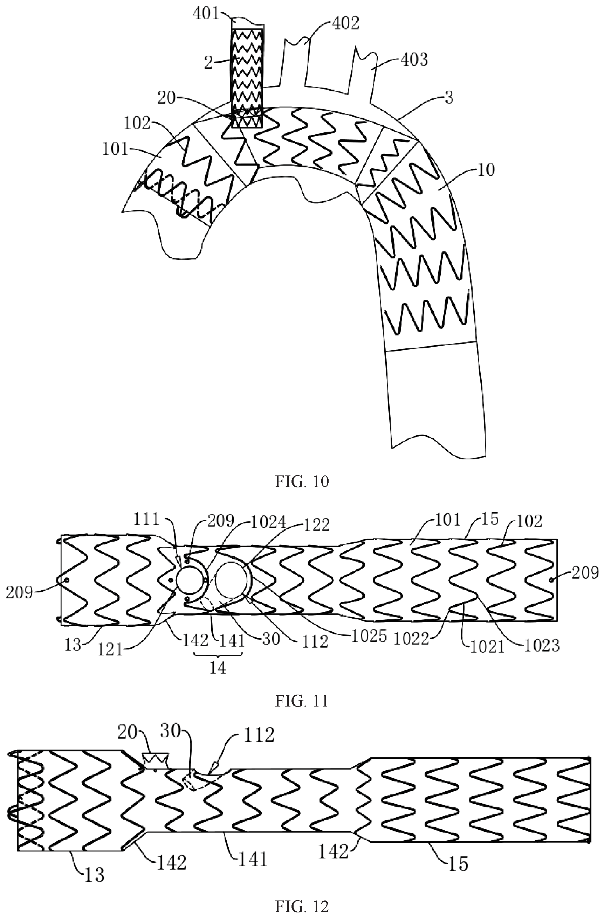

Referring to FIG. 10, in an exemplary application scenario of a stent graft 1, for example, a branch vessel 4 on an aortic arch 3 is reconstructed, and the connecting stent 20 is in the second configuration. The branch vessel 4 is any one of a brachiocephalic artery 401, a left common carotid artery 402, and a left subclavian artery 403. The stent graft 1 is released in the aortic arch 3 through the interventional sheath tube, and the branch stent 2 is released in the connecting stent 20. The states of the main stent 10, the connecting stent 20, and the branch stent 2 after being released in the aortic arch 3 are as illustrated in FIG. 10.

A stent graft is provided in a second embodiment of the present disclosure. Reference can be made to FIG. 11 and FIG. 12, where FIG. 11 is a schematic structural view of a stent graft 1 in a second embodiment (the main support frame 102 located on the inner peripheral wall of the proximal end of the main stent 10 is not shown), and FIG. 12 is a schematic structural view of the stent graft 1 in FIG. 11 from another perspective. The second embodiment of the present disclosure may adopt any possible implementation of the above-described embodiment. Meanwhile, the structure of the stent graft 1 in the second embodiment of the present disclosure may further have the following implementations.

The stent graft 1 includes a main stent 10, a connecting stent 20, and at least one anchor stent 30. Both the connecting stent 20 and the anchor stent 30 are used for being insert-fitted with the branch stent 2. In some implementations, the implementation of the main stent 10 and the connecting stent 20 is the same as any possible implementation of the implementation described above, and will not be repeated herein. Exemplarily, the anchor stent 30 includes an internal graft (not shown) and at least one support ring (not shown) fixed to the internal graft. The structure of the support ring is the same as the structure of any main support frame 102, and will not be described here.

The connecting stent 20 in the first configuration is located outside the main stent 10. The main stent 10 defines at least two fenestrations 11 on the sidewall of the main stent 10. The at least two fenestrations 11 are sequentially defined at intervals from the proximal end of the main stent 10 to the distal end of the main stent 10. The two fenestrations 11 are in communication with the lumen of the main stent 10.

One end of the connecting stent 20 and one end of the anchor stent 30 are respectively fixed to the two fenestrations 11, so that the connecting stent 20 and the anchor stent 30 are respectively in communication with the lumen of the main stent 10 through the two fenestrations 11. In other words, one end of the connecting stent 20 is fixed to either of the two fenestrations 11, and the connecting stent 20 is in communication with the lumen of the main stent 10 through either of the two fenestrations 11; and the anchor stent 30 is fixed to the other fenestration 11 and is in communication with the lumen of the main stent 10 through the other fenestration 11.

For ease of distinction, at least two fenestrations 11 are respectively designated as a first fenestration 111 and a second fenestration 112. The first fenestration 111 is closer to the proximal end of the main stent 10 than the second fenestration 112. A length of the second fenestration 112 in the axial direction of the main stent 10 is larger than a length of the first fenestration 111 in the axial direction of the main stent 10. In other words, from the proximal end of the main stent 10 to the distal end of the main stent 10, the lengths of the fenestrations 11 in the axial direction of the main stent 10 increase sequentially, for example, the length of the first fenestration 111 in the axial direction of the main stent 10 and the length of the second fenestration 112 in the axial direction of the main stent 10 increase sequentially.

In some implementations, the at least two fenestrations 11 on the main stent 10 are defined at intervals in the axial direction of the main stent. An axial distance between edges of two adjacent fenestrations 11 at the proximal end of the main stent 10 ranges from 2 mm to 15 mm. For example, an axial distance between a distal end side of the first fenestration 111 and a proximal end side of the second fenestration 112 ranges from 2 mm to 15 mm, so that the distance between the first fenestration 111 and the second fenestration 112 is relatively close.

In some implementations, the at least two fenestrations 11 on the main stent 10 are not defined at intervals in the axial direction of the main stent 10, that is, the at least two fenestrations 11 may have at least partial overlap in the axial direction of the main stent 10.

The release process of the main stent 10 from the interventional sheath tube is typically achieved by withdrawing the interventional sheath tube to release the main stent 10, that is, by withdrawing the interventional sheath tube, the main stent 10 is exposed relative to the inner cavity of the interventional sheath tube. In the process of withdrawing the interventional sheath tube, the proximal end of the main stent 10 is usually released first, and at this time, the first fenestration 111 closer to the proximal end of the main stent 10 may first be aligned with a root opening of the corresponding branch vessel 4. Therefore, it can be understood that once the alignment between the first fenestration 111 and the root opening of the corresponding branch vessel 4 is determined, the position of the main stent 10 in the trunk vessel is basically determined. On condition that a distance between center positions of the two fenestrations 11 is given, the length of the second fenestration 112 in the axial direction of the main stent 10 is set to be larger, so that the edge of the second fenestration 112 is closer to the edge of the first fenestration 111, which is conducive to improving the fault tolerance rate of the second fenestration 112. In this way, when the main stent 10 is released in the trunk vessel, it is unnecessary to align a center line of the second fenestration 112 precisely with a center line of the target branch vessel 4, thereby reducing the requirement for interventional treatment technology of the operator, and reducing the operation time. Meanwhile, by setting the second fenestration 112 to have larger length in the axial direction of the main stent 10 and to be closer to the first fenestration 111, super-selective positioning is facilitated. Further, by setting the second fenestration 112 to have larger length in the axial direction of the main stent 10 and to be closer to the first fenestration 111, the wider application range is achieved, so that different distances between two adjacent branch vessels 4 on the trunk vessel of different patients can be adapted.

A length of the second fenestration 112 in the circumferential direction of the main stent 10 is larger than a length of the first fenestration 111 in the circumferential direction of the main stent 10. In other words, in the axial direction of the main stent 10, the lengths of the fenestration 11 in the circumferential direction of the main stent 10 increase sequentially, for example, the length of the first fenestration 111 in the circumferential direction of the main stent 10 and the length of the second fenestration 112 in the circumferential direction of the main stent 10 increase sequentially.

The length of the second fenestration 112 in the circumferential direction of the main stent 10 is at least 50%-70% of the diameter of the main stent 10. Therefore, while ensuring the supporting performance of the main stent 10 in the vessel, the fault tolerance rate of the second fenestration 112 is further improved, and the super-selective positioning is more convenient. It is of course ideal that the multiple branch vessels 4 on the trunk vessel are located on the same axial straight line. However, some patients have tortuous trunk vessels with more complex anatomical configurations, where multiple branch vessels 4 on the trunk vessel cannot be located on the same axial straight line. By setting the length of the second fenestration 112 in the circumferential direction of the main stent 10 to be larger, it is possible to achieve reconstruction of the branch vessels 4 under different vascular anatomical configurations, so that the application range is wider.

In some implementations, the connecting stent 20 is closer to the proximal end of the main stent 10 than the anchor stent 30. The connecting stent 20 is fixed to the first fenestration 111. The at least one anchor stent 30 is fixed to the second fenestration 112. The at least one anchor stent 30 is disposed on the second fenestration 112, so that the super-selective positioning of the second fenestration 112 is facilitated, thereby saving operation time and reducing the occurrence of complications.

In some implementations, the at least one anchor stent 30 is located in the lumen of the main stent 10. A radial dimension of the anchor stent 30 gradually decreases from one end of the anchor stent 30 close to the fenestration 11 to one end of the anchor stent 30 away from the fenestration 11, so that the anchor stent 30 has a gradually tapered shape. The end of the anchor stent 30 having a smaller radial dimension helps to improve the connection reliability between the anchor stent 30 and the branch stent 2. In addition, the end of the anchor stent 30 having a larger radial dimension helps to improve the fault tolerance rate of the fenestration 11 where the anchor stent 30 is located, and facilitates the super-selection positioning, so that the application range is wider.

In some implementations, one end of the anchor stent 30 away from the fenestration 11 where the anchor stent 30 is located extends towards the proximal end of the main stent 10, so that a port of the anchor stent 30 away from the fenestration 11 where the anchor stent 30 is located faces towards the proximal end of the main stent 10.

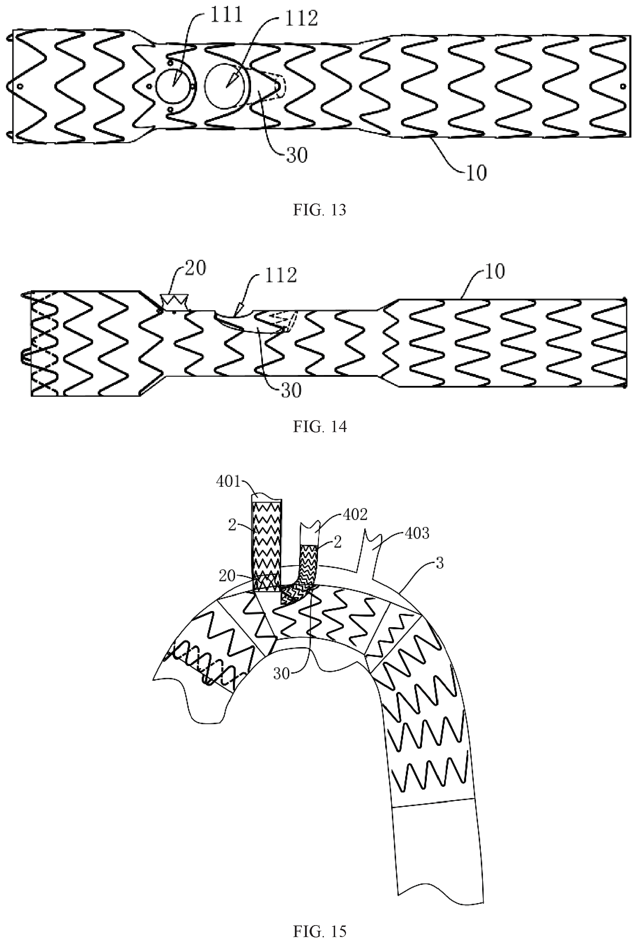

In some implementations, reference can be made to FIG. 13 and FIG. 14, where FIG. 13 is a schematic structural view of a stent graft 1 in some implementations (e.g., the main support frame 102 located on the inner peripheral wall of the proximal end of the main stent 10 may not be shown), and FIG. 14 is a schematic structural view of the stent graft 1 in FIG. 13 from another perspective. One end of the anchor stent 30 away from the fenestration 11 where the anchor stent 30 is located extends towards the distal end of the main stent 10, so that a port of the anchor stent 30 away from the fenestration 11 where the anchor stent 30 is located faces towards the distal end of the main stent 10.

In some implementations, a central axis of the anchor stent 30 is parallel to the central axis of the main stent 10. Alternatively, the central axis of the anchor stent 30 and the central axis of the main stent 10 define an angle therebetween, so that an axial direction of the anchor stent 30 is inclined relative to the axial direction of the main stent 10.

In some implementations, the central axis of the anchor stent 30 is perpendicular to the central axis of the main stent 10. The end of the anchor stent 30 away from the fenestration 11 where the anchor stent 30 is located extends towards the lumen of the main stent 10 along the centerline of the fenestration 11 where the anchor stent 30 is located.

In some implementations, the main stent 10 includes a first support segment 13, a recessed segment 14, and a second support segment 15 extending sequentially from the proximal end of the main stent 10 to the distal end of the main stent 10. A radial dimension of the recessed segment 14 is smaller than each of a radial dimension of the first support segment 13 and a radial dimension of the second support segment 15, so that the recessed segment 14 is at least partially recessed in the radial direction of the main stent 10. For example, the radial dimension of the first support segment 13 is larger than or equal to each of the radial dimension of the recessed segment 14 and the radial dimension of the second support segment 15, and the radial dimension of the recessed segment 14 is smaller than the radial dimension of the second support segment 15, so that the recessed segment 14 is at least partially recessed in the radial direction of the main stent 10.

In some implementations, at least two fenestrations 11 are located on the recessed segment 14. It can be understood that the recessed segment 14 may have its entire circumferential sidewall recessed towards the lumen of the main stent 10 relative to the first support segment 13 and the second support segment 15. Alternatively, the recessed portion 14 may also have a partial circumferential sidewall recessed, for example, one side of the recessed portion 14 where the fenestration 11 is defined may be recessed towards the lumen of the main stent 10.

In some other implementations, at least one fenestration 11 is located on the first support segment 13.

In some other implementations, at least one fenestration 11 is located on the first support segment 13 and at least one fenestration is located on the recessed segment 14.

Exemplarily, the recessed segment 14 includes an avoidance segment 141 and at least one flared portion 13. The at least one flared portion 13 is fixed to a proximal end of the avoidance segment 141. The at least one flared portion 142 extends from the proximal end of the avoidance segment 141 towards the proximal end of the main stent 10. The recessed segment 14 is fixed to a distal end of the first support segment 13 through a proximal end of the flared portion 142.

In some other implementations, the recessed segment 14 includes an avoidance segment 141 and at least one flared portion 142. The at least one flared portion 142 is fixed to the distal end of the avoidance segment 141. The at least one flared portion 142 extends from the distal end of the avoidance segment 141 towards the distal end of the main stent 10. The recessed segment 14 is fixed to a proximal end of the second support segment 15 through a distal end of the flared portion 142.

Exemplarily, the recessed segment 14 includes an avoidance segment 141 and two flared portions 142. The two flared portions 142 are respectively located at both ends of the avoidance segment 141 in the axial direction of the avoidance segment 141. The recessed segment 14 is respectively fixed to the distal end of the first support segment 13 and the proximal end of the second support segment 15 by the two flared portions 142. A radial dimension of the flared portion 142 is larger than the radial dimension of the other regions of the recessed segment 14.

In some implementations, the radial dimension of the flared portion 142 gradually increases from one end of the flared portion 142 close to the avoidance segment 141 towards one end of the flared portion 142 away from the avoidance segment 141, so that the flared portion 142 has a tapered shape.

In some other implementations, in the circumferential direction of the flared portion 142, the flared portion 142 is at least partially enlarged radially in a direction away from the central axis of the recessed segment 14. Exemplarily, in the circumferential direction of the entire flared portion 142, the flared portion 142 is radially enlarged in the direction away from the central axis of the recessed portion 14, so that the sidewall of the avoidance portion 141 in the entire circumferential direction of the avoidance portion 141 may be recessed towards the lumen of the main stent 10 relative to the first support segment 13 and the second support segment 15. In other words, the radial dimension of the flared portion 142 gradually increases from one end of the flared portion 142 close to the middle portion of the recessed portion 14 in the axial direction of the recessed portion 14 to one end of the flared portion 142 away from the middle portion of the recessed portion 14 in the axial direction of the recessed portion 14.

In some other implementations, in the circumferential direction of a part of the flared portion 142, the part of the flared portion 142 is radially enlarged in the direction away from the central axis of the recessed portion 14, so that the circumferential sidewall of the part of the avoidance portion 141 of the recessed portion 14 has a recessed shape. For example, the side of the recessed portion 14 where the fenestration 11 is defined may be recessed towards the lumen of the main stent 10.

In some other implementations, the first fenestration 111 is located on the flared portion 142 close to the proximal end of the main stent 10. When the axial length of the first support segment 13 of the main stent 10 on the proximal end of the body stent 10 relative to the first fenestration 111 is longer, the anchoring length of the main stent 10 is longer, so that the main stent 10 can be more stably supported in the trunk vessel and less prone to axial displacement. By defining the first fenestration 111 on the flared portion 142, the anchoring length of the main stent 10 can be extended.

In some implementations, an edge of a proximal end of the first fenestration 111 is adjacent to an edge of the distal end of the flared portion 142 adjacent to the first support segment 13, which facilitates extending the anchoring length of the main stent 10.

In some implementations, the first fenestration 111 and the second fenestration 112 are located on the avoidance segment 141.

The main stent 10 includes a main covering membrane 101 and multiple main support frames 102 fixed to the main covering membrane 101. The multiple main support frames 102 are arranged at intervals in the axial direction of the main covering membrane 101. At least two fenestrations 11 are defined on the sidewall of the main covering membrane 101. The flared portion 142 is provided with at least one main support frame 102 that changes with the shape of the flared portion 142.

In some implementations, the proximal end of the main stent 10 is provided with two overlapping main support frames 102, which are respectively fixed to the inner peripheral wall of the main covering membrane 101 and the outer peripheral wall of the main covering membrane 101. The crests 1022 and troughs 1023 of the two main support frames 102 overlapping at the proximal end of the main stent 10 are arranged opposite to each other in the axial direction of the main stent 10 (it can be understood that the axial relative arrangement is not strict, and there may be an error of 5%-15%). The support rods 1021 of the two main support frames 102 at the proximal end of the main stent 10 cross and overlap through the main covering membrane 101 in between, serving to enhance the radial support of the proximal end of the main stent 10, thereby improving the wall apposition of the proximal end of the main stent 10 and helping to prevent or reduce axial displacement of the main stent 10 along the trunk vessel.

In one or more embodiments, at least a part of the crest 1022 of at least one main support frame 102 located at the proximal end of the main stent 10 extends beyond the proximal end of the main covering membrane 101, that is, at least a part of the support rod 1021 of the main support frame 102 has a local axial length not covered by the main covering membrane 101.

In some implementations, for a main support frame 102 at at least one side of the first fenestration 111 in the axial direction of the main stent 10, two adjacent support rods 1021 of the main support frame 102 in the circumferential direction of the main stent 10 define a surrounding angle 1024 therebetween. The surrounding angle 1024 and the two adjacent support rods 1021 defining the surrounding angle 1024 cooperatively form a semi-annular structure, and the semi-annular structure at least partially surrounds a first fenestration 111.

In some implementations, for a main support frame 102 at at least one side of the second fenestration 112 in the axial direction of the main stent 10, two adjacent support rods 1021 of the main support frame 102 in the circumferential direction of the main stent 10 define a wall apposition angle 1025 therebetween. The wall apposition angle 1025 and the two adjacent support rods 1021 defining the wall apposition angle 1025 cooperatively form a semi-annular structure, and the semi-annular structure at least partially surrounds a second fenestration 112. For example, in the axial direction of the main stent 10, at least one main support frame 102 is provided between the first fenestration 111 and the second fenestration 112 to improve the radial supporting force around the first fenestration 111 and the second fenestration 112, which is conducive to improving the wall apposition of the first fenestration 111 and the second fenestration 112. For the main support frame 102 at both sides of the second fenestration 112 in the axial direction of the main stent 10, an angle defined between two adjacent support rods 1021 in the circumferential direction of the second fenestration 112 is a wall apposition angle 1025, and the wall apposition angle 1025 is configured as a rounded angle. It can be understood that the wall apposition angle 1025 and the two support rods defining the wall apposition angle 1025 that are located at the proximal end side of the second fenestration 112 may be a surrounding angle 1024 of a semi-annular structure that is defined by enclosing and is at least partially surrounds the first fenestration 111.

In some implementations, the two fenestrations 11 are respectively fixed with a support ring 12 for reinforcing the configuration of the fenestrations 11. For ease of distinction, at least two fenestrations 11 are respectively designated as a first fenestration 111 and a second fenestration 112. The first fenestration 111 is fixed with a first support ring 121 for reinforcing the configuration of the first fenestration 111.

In some implementations, the second fenestration 112 is fixed with a second support ring 122 for reinforcing the configuration of the second fenestration 112.

In some implementations, two ends of the second support ring 122 in the axial direction of the main stent 10 are raised, that is, the two ends of the second support ring 122 in the axial direction of the main stent 10 are raised compared with other regions of the second support ring 122, which is conducive to lifting the main covering membrane 101 and the internal graft in a direction away from the central axis of the main stent 10. Therefore, the lumen loss rate of the main stent 10 can be further reduced, more blood can flow through the lumen of the main stent 10, and more blood can flow to the branch vessel 4 through the second fenestration 112, thereby reducing the occurrence of complications.

In some other implementations, two ends of the first support ring 121 in the axial direction of the main stent 10 are raised, that is, the two ends of the first support ring 121 in the axial direction of the main stent 10 are raised compared with other regions of the first support ring 121, which is conducive to lifting the main covering membrane 101 and the internal graft in a direction away from the central axis of the main stent 10. Therefore, the lumen loss rate of the main stent 10 can be further reduced, more blood can flow through the lumen of the main stent 10, and more blood can flow to the branch vessel 4 through the first fenestration 111, thereby reducing the occurrence of complications.

In some implementations, the main stent 10 further includes a radiopaque structure 209. The radiopaque structure is disposed around at least one of the first fenestration 111 or the second fenestration 112, and is used for indicating a position of at least one of the first fenestration 111 or the second fenestration 112. Exemplarily, the radiopaque structure 209 includes two annular structures respectively disposed around the first fenestration 111 and the second fenestration 112.

In other implementations, the first fenestration 111 and the second fenestration 112 may have a radiopaque material. The radiopaque structure 209 at least partially surrounds the proximal end of the main stent 10; or the radiopaque structure 209 is at least partially disposed around the distal end of the main stent 10.

Referring to FIG. 15, in an exemplary application scenario of a stent graft 1, for example, two branch vessels 4 on the aortic arch 3 are reconstructed, and the connecting stent 20 is in the first configuration. The two branch vessels 4 are the brachiocephalic artery 401 and the left common carotid artery 402, respectively. The stent graft 1 is released in the aortic arch 3 through the interventional sheath tube, and the branch stent 2 is respectively released in the connecting stent 20 and the anchor stent 30. The states of the main stent 10, the connecting stent 20, the anchor stent 30, and the branch stent 2 after being released in the aortic arch 3 are illustrated in FIG. 15.

In some other implementations, the two branch vessels 4 are the left common carotid artery 402 and the left subclavian artery, respectively. In some other implementations, the two branch vessels 4 are the brachiocephalic artery 401 and the left subclavian artery, respectively.

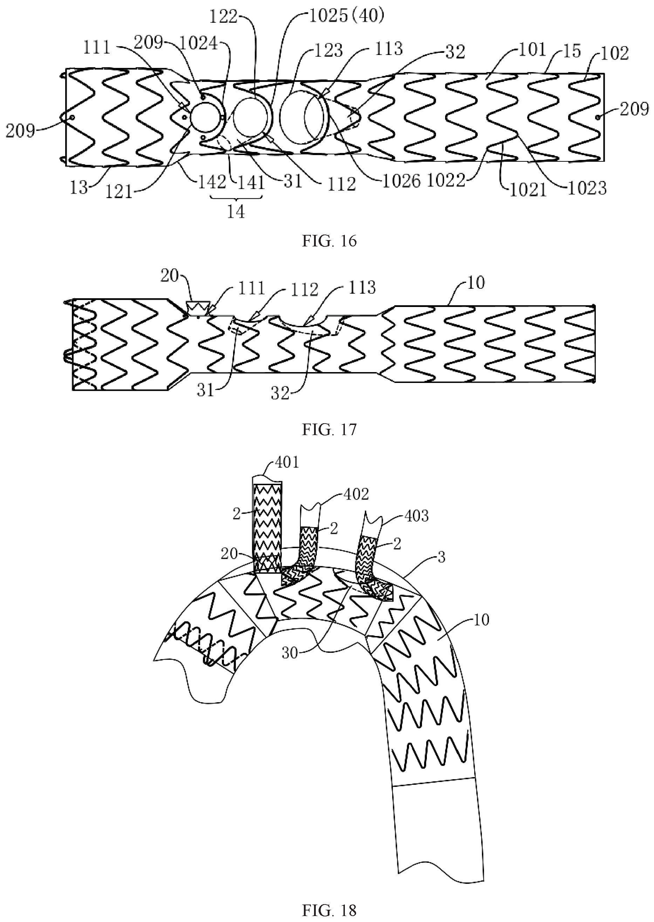

A stent graft is provided in a third embodiment of the present disclosure. Reference can be made to FIG. 16 and FIG. 17, where FIG. 16 is a schematic structural view of a stent graft 1 in a third embodiment, and FIG. 17 is a schematic structural view of the stent graft in FIG. 16 from another perspective. The third embodiment of the present disclosure can adopt any implementation of any embodiment described above. Meanwhile, the stent graft 1 in the third embodiment of the present disclosure defines at least three fenestrations 11. The stent graft 1 includes a main stent 10, a connecting stent 20, and at least two anchor stents 30. Both the connecting stent 20 and the anchor stent 30 are used to being insert-fitted with the branch stent 2. The branch stent 2 is used to be released in the branch vessel 4 to reconstruct the branch vessel 4. The structure, shape, position, etc., of the main stent 10 and the connecting stent 20 adopt any possible implementation of the connecting stent 20 of any embodiment described above, and will not be repeated herein. The main stent 10 defines at least three fenestrations 11 on the sidewall of the main stent 10. The at least three fenestrations 11 are sequentially arranged at intervals from the proximal end of the main stent 10 to the distal end of the main stent 10. The three fenestrations 11 are in communication with the lumen of the main stent 10.

For ease of distinction, at least two anchor stents 30 are designated as a first stent 31 and a second stent 32, respectively. The structure, shape, position, etc., of the first stent 31 and the second stent 32 adopt any possible implementation of the anchor stent 30 or the connecting stent 20 of any embodiment described above, and will not be repeated herein.

In some implementations, one end of the connecting stent 20, one end of the first stent 31, and one end of the second stent 32 are fixed to the three fenestrations 11, respectively, so that the connecting stent 20, the first stent 31, and the second stent 32 are in communication with the lumen of the main stent 10 through the three fenestrations 11, respectively. In other words, one end of the connecting stent 20 is fixed to any one of the three fenestrations 11, and the connecting stent 20 is in communication with the lumen of the main stent 10 through any one of the three fenestrations 11; and the two anchor stents 30 (i.e., the first stent 31 and the second stent 32) are respectively fixed to the other two fenestrations 11, and are in communication with the lumen of the main stent 10 through the corresponding fenestration 11.

In some implementations, the connecting stent 20, the first stent 31, and the second stent 32 are fixed to the three fenestrations 11 from the proximal end of the main stent 10 to the distal end of the main stent 10, respectively. One end of the second stent 32 away from one end of the second stent 32 where the fenestration 11 is located extends towards the distal end of the main stent 10, so that a port of the second stent 32 away from the end of the fenestration 11 where the second stent 32 is located faces towards the distal end of the main stent 10.

In some implementations, at least one of two ports of the second stent 32 in the axial direction of the second stent 32 is configured as an oblique port. An oblique-port surface of the second stent 32 away from the end of the second stent 32 where the fenestration 11 is fixed faces away from a surface of the second stent 32 where the fenestration 11 is fixed, thereby facilitating the super-selective positioning. In other words, one end of the second stent 32 away from the end of the second stent 32 where the fenestration 11 is located is configured as an oblique port, and one side of the oblique port close to the end of the second stent 32 where the fenestration 11 is located is farther from the distal end of the main stent 10 than one side of the oblique port away from the end of the second stent 32 where the fenestration 11 is located.

For ease of distinction, the at least three fenestrations are designated as a first fenestration 111, a second fenestration 112, and a third fenestration 113, respectively. The first fenestration 111 is closer to the proximal end of the main stent 10 than the second fenestration 112. The second fenestration 112 is closer to the proximal end of the main stent 10 than the third fenestration 113. The length of the third fenestration 113 in the axial direction of the main stent 10 is larger than the length of the second fenestration 112 in the axial direction of the main stent 10. The length of the second fenestration 112 in the axial direction of the main stent 10 is larger than the length of the first fenestration 111 in the axial direction of the main stent 10. In other words, from the proximal end to the distal end of the main stent 10, the lengths of the fenestrations in the axial direction of the main stent 10 increase sequentially, for example, the length of the first fenestration 111 in the axial direction of the main stent 10, the length of the second fenestration 112 in the axial direction of the main stent 10, and the length of the third fenestration 113 in the axial direction of the main stent 10 increase sequentially.

In some implementations, at least two fenestrations 11 on the main stent 10 are arranged at intervals in the axial direction of the main stent. An axial distance between the edges of two adjacent fenestrations at the proximal end of the main stent 10 ranges from 2 mm to 15 mm. For example, an axial distance between a distal end side of the first fenestration 111 and a proximal end side of the second fenestration 112 ranges from 2 mm to 15 mm, so that the distance between the first fenestration 111 and the second fenestration 112 is relatively close.

In some implementations, at least three fenestrations 11 on the main stent 10 are arranged at intervals along the axial direction of the main stent. The axial distance between the edges of any two adjacent fenestrations is 2 mm-15 mm, for example, the axial distance between the distal end side of the first fenestration 111 and the proximal end side of the second fenestration 112 is 2 mm-15 mm, so that the distance between the first fenestration 111 and the second fenestration 112 is relatively close; The axial distance between the distal end side of the second fenestration 112 and the proximal end side of the third fenestration 113 is 2 mm-15 mm, so that the distance between the second fenestration 112 and the third fenestration 113 is relatively close.

In some implementations, the at least two fenestrations 11 on the main stent 10 are not defined at intervals in the axial direction of the main stent 10, that is, the at least two fenestrations 11 may have at least partial overlap in the axial direction of the main stent 10.

On condition that a distance between a center position of the first fenestration 111 and a center position of the second fenestration 112 is given, the length of the second fenestration 112 in the axial direction of the main stent 10 is set to be larger, so that an edge of the second fenestration 112 at the proximal end side can be closer to an edge of the first fenestration 111 at the distal end side, which is conducive to improving the fault tolerance rate of the second fenestration 112. In this way, when the main stent 10 is released in the trunk vessel, it is unnecessary to align a central line of the second fenestration 112 precisely with a central line of the target branch vessel 4, thereby reducing the requirement for interventional treatment technology of the operator, and reducing the operation time. Meanwhile, by setting the second fenestration 112 to have larger length in the axial direction of the main stent 10 than the first fenestration 111 and to be closer to the first fenestration 111, the super-selective positioning is more facilitated. Further, by setting the second fenestration 112 to have larger length in the axial direction of the main stent 10 than the first fenestration 111 and to be closer to the first fenestration 111, the wider application range is achieved, so that different distances between two adjacent branch vessels 4 on the trunk vessel of different patients can be adapted.

Similarly for the second fenestration 112 and the third fenestration 113, on condition that the release has been completed at positions where the first fenestration 111 and the second fenestration 112 are located, the length of the third fenestration 113 in the axial direction of the main stent 10 is set to be larger, so that an edge of the third fenestration 113 at the proximal end side is closer to an edge of the second fenestration 112 at the distal end side, which is conducive to improving the fault tolerance rate of the third fenestration 113. In this way, when the main stent 10 is released in the trunk vessel, it is unnecessary to align a center line of the third fenestration 113 precisely with the center line of the target branch vessel 4, thereby reducing the requirement for interventional treatment technology of the operator, and reducing the operation time. Meanwhile, by setting the third fenestration 113 to have larger length in the axial direction of the main stent 10 than the second fenestration 112 and to be closer to the second fenestration 112, the super-selective positioning is more facilitated. Further, by setting the third fenestration 113 to have larger length in the axial direction of the main stent 10 than the second fenestration 112 and to be closer to the second fenestration 112, the wider application range is achieved, so that different distances between two adjacent branch vessels 4 on the trunk vessel of different patients can be adapted.

In some implementations, the length of the second fenestration 112 in the circumferential direction of the main stent 10 is at least 50%-70% of the diameter of the main stent 10. Therefore, while ensuring the supporting performance of the main stent 10 in the vessel, the fault tolerance rate of the second fenestration 112 is improved, and the super-selection positioning is more convenient, so that the application range is wider.

In some implementations, the lengths of at least three fenestrations in the circumferential direction of the main stent 10 increase sequentially from the proximal end of the main stent 10 to the distal end of the main stent 10. For example, the length of the first fenestration 111 in the circumferential direction of the main stent 10, the length of the second fenestration 112 in the circumferential direction of the main stent 10, and the length of the third fenestration 113 in the circumferential direction of the main stent 10 increase sequentially.

In some implementations, the length of the second fenestration 112 in the circumferential direction of the main stent 10 is larger than the length of the first fenestration 111 in the circumferential direction of the main stent 10.

In some implementations, the length of the third fenestration 113 in the circumferential direction of the main stent 10 is larger than the length of the second fenestration 112 in the circumferential direction of the main stent 10. The length of the third fenestration 113 in the circumferential direction of the main stent 10 is at least 60%-90% of the diameter of the main stent 10. Therefore, while ensuring the supporting performance of the main stent 10 in the vessel, the fault tolerance rate of the third fenestration 113 is improved, and the super-selection positioning is more convenient, so that the reconstruction of the branch vessel 4 under different vascular anatomical configurations is achieved in a wider range, and the application range is wider.

In some implementations, for a main support frame 102 located at at least one side of the first fenestration 111 in the axial direction of the main stent 10, two adjacent support rods 1021 of the main support frame 102 in the circumferential direction of the main stent 10 define a surrounding angle 1024 therebetween. The surrounding angle 1024 is configured as a rounded angle.

In some implementations, for a main support frame 102 at at least one side of the second fenestration 112 in the axial direction of the main stent 10, two adjacent support rods 1021 of the main support frame 102 in the circumferential direction of the main stent 10 define a wall apposition angle 1025 therebetween, and the wall apposition angle 1025 is configured as a rounded angle. For example, at the distal end side of the second fenestration 112, the wall apposition angle 1025 and the two support rods 1021 defining the wall apposition angle 1025 cooperatively form a semi-annular structure, and the semi-annular structure at least partially surrounds a second fenestration 112. For example, in the axial direction of the main stent 10, at least one main support frame 102 is provided between the first fenestration 111 and the second fenestration 112 to improve the radial supporting force around the first fenestration 111 and the second fenestration 112, which is conducive to improving the wall apposition of the first fenestration 111 and the second fenestration 112. For a main support frame 102 at both sides of the second fenestration 112 in the axial direction of the main stent 10, an angle defined between two adjacent support rods 1021 in the circumferential direction of the main stent 10 is the wall apposition angle, and the apposition angle 1025 is configured as a rounded angle.

It can be understood that the wall apposition angle 1025 and the two support rods defining the wall apposition angle 1025 that are located at the proximal end side of the second fenestration 112 may be a surrounding angle 1024 of a semi-annular structure that is defined by enclosing and is at least partially surrounds the first fenestration 111.

In some implementations, at least one of the three fenestrations 11 is fixed with a support ring 12, for example, each of the three fenestrations 11 is fixed with a support ring 12 for reinforcing the configuration of the fenestration 11. Specifically, the first fenestration 111 is fixed with a first support ring 121 for reinforcing the configuration of the first fenestration 111. The second fenestration 112 is fixed with a second support ring 122 for reinforcing the configuration of the second fenestration 112. The third fenestration 113 is fixed with a third support ring 12312 for reinforcing the configuration of the third fenestration 113.

In some implementations, both ends of the second support ring 122 in the axial direction of the main stent 10 are raised, that is, the two ends of the second support ring 122 in the axial direction are raised compared with other regions of the second support ring 122, which is conducive to lifting the main covering membrane 101 and the internal graft in a direction away from the central axis of the main stent 10. Therefore, the lumen loss rate of the main stent 10 can be further reduced, more blood can flow through the lumen of the main stent 10, and more blood can flow to the branch vessel 4 through the second fenestration 112, thereby reducing the occurrence of complications.

In some implementations, for a main support frame 102 located at at least one side of the third fenestration 113 in an axial direction of the third fenestration 113, two adjacent support rods 1021 in a circumferential direction of the third fenestration 113 define a sealing angle 1026 therebetween. The sealing angle 1026 is configured as a rounded angle. For example, at the distal end side of the third fenestration 113, the sealing angle 1026 and the two support rods 1021 defining the sealing angle 1026 cooperatively form a semi-annular structure, and the semi-annular structure at least partially surrounds a third fenestration 113.

The semi-annular structure partially surrounding the first fenestration 111, the semi-annular structure partially surrounding the second fenestration 112, or the semi-annular structure partially surrounding the third fenestration 113 can better fit the configuration of the corresponding fenestration 11, so that the corresponding fenestration 11 better fits against the inner wall of the vessel, which is conducive to improving the sealing performance of the corresponding fenestration 11, thereby enhancing the endoleak prevention effect of the corresponding fenestration 11.

In some implementations, in the axial direction of the main stent 10, at least one main support frame 102 is provided between the second fenestration 112 and the third fenestration 113 to improve the radial supporting force around the second fenestration 112 and the third fenestration 113, which is conducive to improving the wall apposition of the second fenestration 112 and the third fenestration 113. For a main support frame 102 at both sides of the third fenestration 113 in the axial direction of the main stent 10, an angle defined between two adjacent support rods 1021 in the circumferential direction of the main stent 10 is a sealing angle 1026, and the sealing angle 1026 is configured as a rounded angle. It can be understood that the sealing angle 1026 and the two support rods defining the sealing angle 1026 that are located at the proximal end side of the third fenestration 113 may be a wall apposition angle 1025 of a semi-annular structure that is defined by enclosing and is at least partially surrounds the second fenestration 112.

In some implementations, the length of the third fenestration 113 in the circumferential direction of the main stent 10 is larger than the length of the second fenestration 112 in the circumferential direction of the main stent 10. The third fenestration 113 having a larger length in the circumferential direction of the main stent 10 may result in a greater lumen loss rate of the main stent 10. By setting the sealing angle 1026 in the raised shape, it may be conducive to lifting the main covering membrane 101 at the proximal end side and/or the distal end side of the third fenestration 113 in a direction away from the central axis of the main stent 10, thereby reducing the lumen loss rate of the main stent 10, and facilitating more blood flow through the lumen of the main stent 10. Therefore, more blood can flow to the branch vessel 4 through the third fenestration 113, thereby reducing the occurrence of complications.

In some implementations, two ends of the third support ring 12312 in the axial direction of the main stent 10 are raised, that is, the two ends of the third support ring 12312 in the axial direction of the main stent 10 are raised compared with other regions of the third support ring 12312, which is conducive to lifting the main covering membrane 101 and the internal graft in a direction away from the central axis of the main stent 10. Therefore, the lumen loss rate of the main stent 10 can be further reduced, more blood can flow through the lumen of the main stent 10, and more blood can flow to the branch vessel 4 through the third fenestration 113, thereby reducing the occurrence of complications.

In some implementations, in the axial direction of the main stent 10, two main support frames 102 are provided between the second fenestration 112 and the third fenestration 113, and the two main support frames 102 are spaced apart from each other. An angle located at the proximal end side of the third fenestration 113 is the wall apposition angle 1025. The wall apposition angle 1025 located at the proximal end side of the third fenestration 113 and two support rods 1021 defining the wall apposition angle 1025 cooperatively form a semi-annular structure, and the semi-annular structure at least partially surrounds a second fenestration 112. An angle located at the distal end side of the second fenestration 112 is the wall apposition angle 1025. The wall apposition angle 1025 located at the distal end side of the second fenestration 112 and the two support rods 1021 defining the wall apposition angle 1025 cooperatively form a semi-annular structure, and the semi-annular structure at least partially surrounds the second fenestration 112.

In some implementations, the semi-annular structure located at the proximal end side of the third fenestration 113 and the semi-annular structure located at the distal end side of the second fenestration 112 at least partially overlap in the axial direction of the main stent 10, to form a reinforcing semi-annular structure 40. The reinforcing semi-annular structure 40 at least partially surrounds the second fenestration 112. The overlapping can be understood as that the two semi-annular structures are fixedly connected together; alternatively, the main support frame 102 where one of the two semi-annular structures is located is an open-annular structure with a notch, and the support rod 1021 corresponding to the open-annular structure is fixed on the main support frame 102 where the other of the two semi-annular structures is located, so as to form a semi-annular structure with only one rod body.

In some implementations, the main stent 10 further includes a radiopaque structure 209. The radiopaque structure 209 surrounds at least one of the first fenestration 111, the second fenestration 112, or the third fenestration 113, for indicating a position of at least one of the first fenestration 111, the second fenestration 112, or the third fenestration 113.

In some implementations, the radiopaque structure 209 is a ring-shaped structure surrounding at least one of the first fenestration 111, the second fenestration 112, or the third fenestration 113. In other embodiments, the first fenestration 111, the second fenestration 112, and the third fenestration 113 may have a radiopaque material. The radiopaque structure 209 may also at least partially surround the proximal end of the main stent 10. The radiopaque structure 209 may also at least partially surround the distal end of the main stent 10.

Referring to FIG. 18, in an exemplary application scenario of a stent graft 1, for example, three branch vessels 4 on the aortic arch 3 are reconstructed, and the connecting stent 20 is in the first configuration. The three branch vessels 4 may be the brachiocephalic artery 401, the left common carotid artery 402, and the left subclavian artery, respectively. The stent graft 1 is released in the aortic arch 3 through the interventional sheath tube, the branch stent 2 is released in the connecting stent 20 and the anchor stent 30, respectively. The states of the main stent 10, the connecting stent 20, the anchor stent 30, and the branch stent 2 after being released in the aortic arch 3 are illustrated in FIG. 18.

A stent graft 1 is provided in a fourth embodiment of the present disclosure. The stent graft 1 in the fourth embodiment is different from at least one of the stent graft 1 in the second embodiment or the stent graft 1 in the third embodiment in that, in the fourth embodiment, an anchor stent 30 fixed to the second fenestration 112 is a rotatable stent, and the anchor stent 30 is located in the lumen of the main stent 10.

In some implementations, the central axis of the anchor stent 30 on the second fenestration 112 is perpendicular to the central axis of the main stent 10.

In the fourth embodiment, at least three fenestrations 11 are provided as an example. For ease of distinction, the at least three fenestrations 11 are denoted as a first fenestration 111, a second fenestration 112, and a third fenestration 113, respectively. The first fenestration 111, the second fenestration 112, and the third fenestration 113 are sequentially defined at intervals from the proximal end of the stent graft 1 to the distal end of the stent graft 1. The connecting stent 20 is disposed on the first fenestration 111. In the fourth embodiment, the anchor stent 30 disposed on the second fenestration 112 is named as the first stent 31, and the anchor stent 30 disposed on the third fenestration 113 is named as the second stent 32.

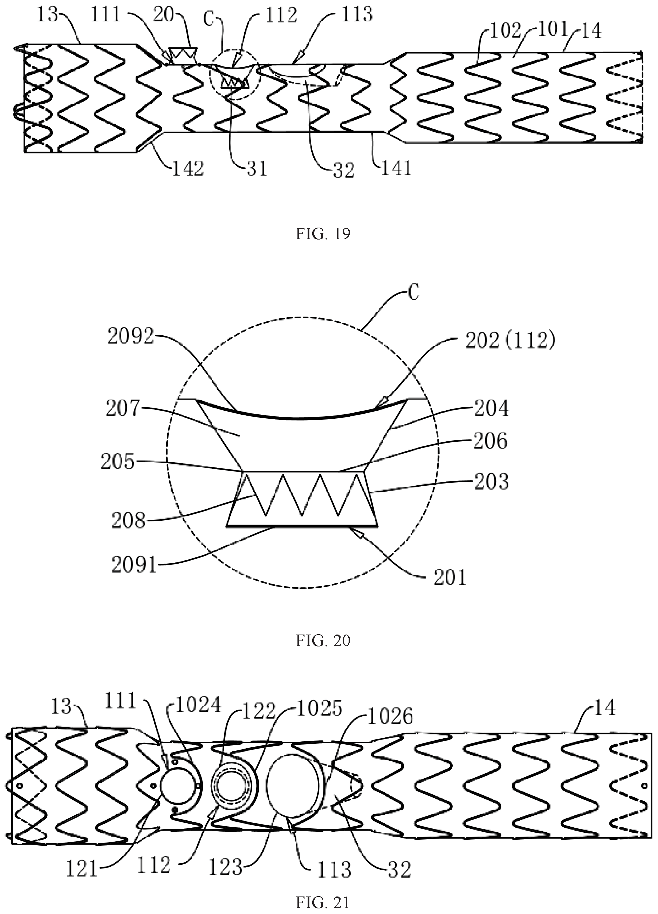

Reference can be made to FIG. 19 to FIG. 21, where FIG. 19 is a schematic structural view of a stent graft 1 in the fourth embodiment, and FIG. 20 is a partial enlarged schematic structural view of FIG. 19 at Circle C (with the main support frame 102 hidden), and FIG. 21 is a schematic structural view of the stent graft 1 illustrated in FIG. 19 from another perspective. The structure, configuration, beneficial effect, or exemplary usage scenario of the first stent 31 can refer to the structure, configuration, beneficial effect, or exemplary usage scenario of the connecting stent 20 at any possible implementation of any embodiment described above, and will not be repeated herein.

In the fourth embodiment, the first stent 31 defines a first opening 201, a second opening 202, and an inner cavity extending between the first opening 201 and the second opening 202. The first opening 201 of the first stent 31 and the second opening 202 of the first stent 31 are located at two ends of the first stent in the axial direction of the first stent. The first stent 31 is fixed to at least one fenestration 11, such as the second fenestration 112, through the end of the first stent 31 where the second opening 202 is located, so that the first stent 31 is in communication with the main stent 10. The first stent 31 includes a first segment 203 and a second segment 204 fixed to the first segment 203. The first opening 201 is located at one end of the first segment 203 away from the second segment 204. The second opening 202 is located at one end of the second segment 204 away from the first segment 203.

Compared with the second segment 204 of the first stent 31, the first segment 203 of the first stent 31 is farther from the fenestration 11 where the first stent 31 is disposed, such as the second fenestration 112. That is, the first stent 31 has a first configuration and a second configuration. The first stent 31 is switchable between the first configuration and the second configuration. The axial length of the first stent 31 in the first configuration is larger than the axial length of the first stent 31 in the second configuration. The first stent 31 in the first configuration is located in the lumen of the main stent 10.

The first stent 31 is fixed to the second fenestration 112 through the second opening 202, so that the second fenestration 112 is in communication with the inner cavity of the first stent 31 and the inner cavity of the main stent 10 through the second opening 202.

In some implementations, the first stent 31 in the second configuration is at least partially located outside the main stent 10.

In some implementations, the cross-sectional dimension of the first opening 201 of the first stent 31 is smaller than the cross-sectional dimension of the second opening 202. Therefore, when the first stent 31 is in the second configuration, in the axial direction of the first stent 31, the first stent 31 at least partially extends in a direction away from the lumen of the main stent 10 out of the fenestration 11 where the first stent 31 is disposed, such as the second fenestration 112. Therefore, it is conducive to further improving the fault tolerance rate of the alignment between the corresponding fenestration 11 (such as the second fenestration 112) and the target vessel opening, reducing the risk of bending of the branch stent 2, and also facilitating the super-selection of the guidewire between the first stent 31 and the target branch vessel.

In some other implementations, the cross-sectional dimension of the first opening 201 of the first stent 31 is larger than or equal to the cross-sectional dimension of the second opening 202, so as to control the extent to which the first stent 31 in the second configuration extends out of the fenestration 112 where the first stent 31 is disposed, for example, to control the extent to which the first stent 31 in the second configuration extends out of the second fenestration 112.

In some implementations, a neck 205 of the first stent 31 is formed at a connection of the first segment 203 and the second segment 204. That is, the first stent 31 forms the first segment 203 and the second segment 204 by the neck 205. When the first stent 31 is in the first configuration, the first segment 203 and the second segment 204 are arranged in the axial direction of the first stent 31 and are in communication with each other.

When the first stent 31 is in the first configuration, the first segment 203 of the first stent 31 is expanded relative to the second segment 204. At this time, the axial length of the first stent 31 is the sum of the length of the first segment 203 and the length of the second segment 204. When the first stent 31 is in the first configuration, the first stent 31 can be located inside the main stent 10, and the first segment 203 is farther from the fenestration 11 where the first stent 31 is disposed, such as the second fenestration 112, than the second segment 204, the first stent 31 being an embedded branch.

In some implementations, the cross-sectional dimension of the first stent 31 at the neck 205 of the first stent 31 is smaller than at least one of the cross-sectional dimension of the first opening 201 of the first stent 311 or the cross-sectional dimension of the second opening 202 of the first stent 311.

In some implementations, the cross-sectional diameter of the neck 205 of the first stent 31 is smaller than each of the cross-sectional diameter of the first opening 201 and the cross-sectional diameter of the second opening 202.

In some implementations, the cross-sectional dimension of one end of the first segment 203 of the first stent 31 close to the second segment 204 of the first stent 31 is smaller than the cross-sectional dimension of one end of the first segment 203 away from the second segment 204, that is, the cross-sectional dimension of one end of the first segment 203 close to the second segment 204 is smaller than the cross-sectional dimension of the first opening 201 of the first stent 31, so that the neck 205 of the first stent 31 is formed at the connection of the first segment 203 and the second segment 204. Alternatively, the cross-sectional dimension of one end of the second segment 204 close to the first segment 203 is smaller than the cross-sectional dimension of one end of the second segment 204 away from the first segment 203, that is, the cross-sectional dimension of the end of the second segment 204 close to the first segment 203 is smaller than the cross-sectional dimension of the second opening 202 of the first stent 31, so that the neck 205 of the first stent 31 is formed at the connection of the first segment 203 and the second segment 204.

In some implementations, the cross-sectional dimension of one end of the first segment 203 of the first stent 31 close to the second segment 204 of the first stent 31 is smaller than the cross-sectional dimension of one end of the first segment 203 away from the second segment 204, and the cross-sectional dimension of one end of the second segment 204 close to the first segment 203 is smaller than the cross-sectional dimension of one end of the second segment 204 away from the first segment 203, so that the neck 205 of the first stent 31 is formed at the connection of the first segment 203 and the second segment 204.

Exemplarily, the first opening 201 of the first stent 31 is located at one end of the first segment 203 away from the second segment 204. In the axial direction of the first stent 31, the cross-sectional diameter of the first segment 203 gradually decreases from one end of the first stent 31 where the first opening 201 is located to the neck 205.

Exemplarily, the second opening 202 is located at one end of the second segment 204 away from the first segment 203. In the axial direction of the first stent 31, the cross-sectional diameter of the second segment 204 gradually decreases from one end of the first stent 31 where the second opening 202 is located to the neck 205.

In some implementations, both the first segment 203 and the second segment 204 are designed to have a tapered shape, which facilitates a closer fitting between the first segment 203 and the second segment 204, so that the branch stent 2 can be more stably inserted into the first stent 31, or in other words, the branch stent 2 can be more stably inserted into the first stent 31 in the second configuration.

In some implementations, the first segment 203 of the first stent 31 and the second segment 204 of the first stent 31 are both in the shape of a truncated cone. The first stent 31 is in the shape of a slender waist, an hourglass, or the like. Compared with the cylindrical structure, the design of the first stent 31 in the present disclosure (for example, the first stent 31 is in the shape of a slender waist or an hourglass) allows the first stent 31 to bend at the neck 205, so that the first stent 31 has expandability, thereby having an extended configuration and a retracted configuration.

In some implementations, either of the first segment 203 of the first stent 31 and the second segment 204 of the first stent 31 is in the shape of a truncated cone, and either of the first segment 203 of the first stent 31 and the second segment 204 of the first stent 31 is in the shape of a taper.

In some implementations, the ratio of the cross-sectional diameter of the first opening 201 or the second opening 202 of the first stent 31 to the cross-sectional diameter of the neck 205 is greater than 1. Exemplarily, the ratio of the cross-sectional diameter of the first opening 201 or the second opening 202 to the cross-sectional diameter of the neck 205 ranges from 1.1 to 2.1.

Exemplarily, the ratio of the cross-sectional diameter of the first opening 201 or the second opening 202 to the cross-sectional diameter of the neck 205 is 1.2. At this time, the expandability of the first stent 31 is good.

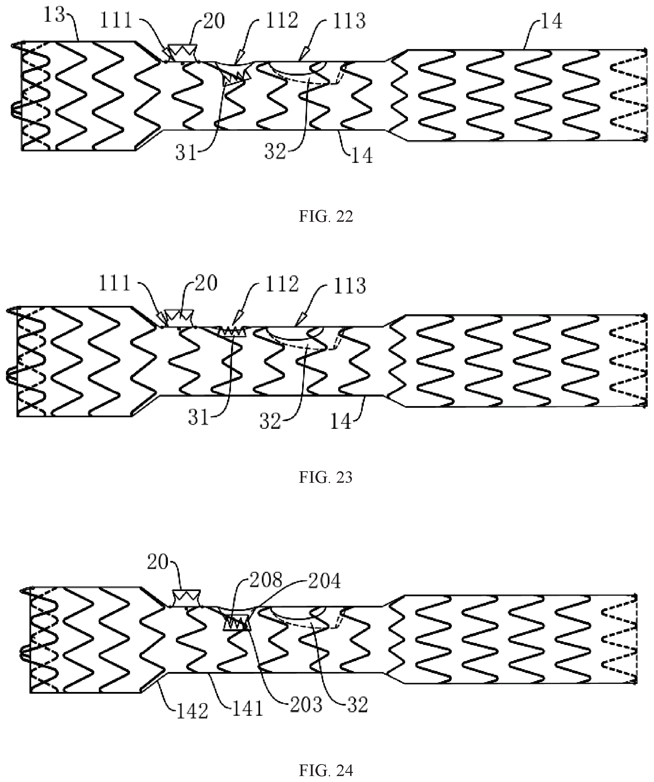

Reference can be made to FIG. 22, which is a rotation schematic view of a first stent 31 in the stent graft 1 in the fourth embodiment in any direction. The second segment 204 of the first stent 31 is a flexible segment, that is, the second segment 204 of the first stent 31 can better assist the 360° rotation of the first stent 31, thereby improving the fault tolerance rate of the alignment between the fenestration 11 corresponding to the branch and the target vessel opening, and reducing the risk of bending of the branch stent 2.

Reference can be made to FIG. 23, which is a schematic structural view of the first stent 31 in the retracted state in the stent graft 1 in the fourth embodiment. The first segment 203 of the first stent 31 is provided with a support member 208 of any implementation described above. The second segment 204 of the connecting stent 20 is a flexible segment formed by the connecting covering membrane 207. The design in which the first segment 203 of the first stent 31 serves as a support anchoring segment and the second segment 204 of the first stent 31 serves as a flexible rotating segment, facilitates the extension and retraction of the first stent 31 in the axial direction of the first stent 31, thereby reducing the volume of the first stent 31 in the lumen of the main stent 10, and reducing the lumen loss rate of the main stent 10. In some other implementations, reference can be made to FIG. 24, which is a schematic structural view of a first stent 31 in a stent graft 1 in other implementations. For the first stent 31, the support member 208 on the first segment 203 axially extends towards the second segment 204, so that at least a part of the second segment 204 has a supporting effect, thereby increasing the anchoring force between the connecting stent 20 and the branch stent 2, and thus improving the connection firmness between the connecting stent 20 and the branch stent 2.

In some implementations, the axial length of the connecting stent 20 in the extended configuration is larger than the axial length of the first stent 31 in the extended state. This arrangement is advantageous for increasing the rotation range of the connecting stent 20, so that even if when the space of the trunk vessel and the branch vessel 4 is narrow, the connecting stent 20 can still be positioned to the root opening of the target branch vessel 4, thereby reducing the impact on the long-term patency of the corresponding branch stent 2.

In some implementations, the length of the second fenestration 112 in the circumferential direction of the main stent 10 is larger than the length of the second fenestration 112 in the axial direction of the main stent 10, so that the second fenestration 112 presents a "transversely oriented" elliptical shape. This arrangement is advantageous for improving the positioning fault tolerance rate of the second fenestration 112 in the circumferential direction of the main stent 10, better adapting to different positions of the branch vessel 4 relative to the trunk vessel, and providing a wider adaptation range.

In some implementations, the length of the third fenestration 113 in the circumferential direction of the main stent 10 is larger than the length of the third fenestration 113 in the axial direction of the main stent 10, so that the third fenestration 113 presents a "transversely oriented" elliptical shape. This arrangement is advantageous for improving the positioning fault tolerance rate of the third fenestration 113 in the circumferential direction of the main stent 10, better adapting to different positions of the branch vessel 4 relative to the trunk vessel, and providing a wider adaptation range.

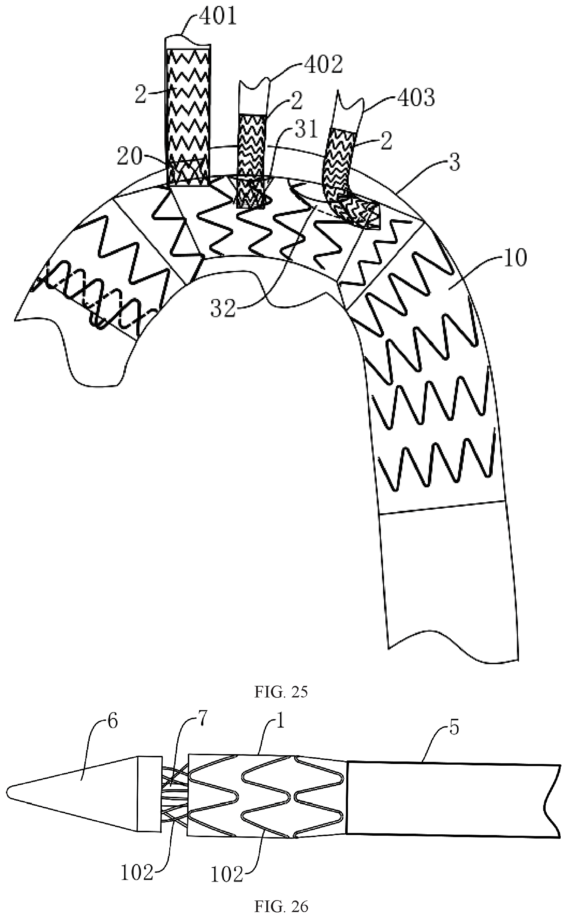

Referring to FIG. 25, in an exemplary application scenario of a stent graft 1, for example, three branch vessels 4 on the aortic arch 3 are reconstructed, the connecting stent 20 is in the extended configuration, and the first stent 31 is in the extended state. The three branch vessels 4 are the brachiocephalic artery 401, the left common carotid artery 402, and the left subclavian artery, respectively. The stent graft 1 is released in the aortic arch 3 through the interventional sheath tube. The branch stent 2 is released in the connecting stent 20, the first stent 31, and the second stent 32, respectively. The states of the main stent 10, the connecting stent 20, the first stent 31, the second stent 32, and the branch stent 2 after being released in the aortic arch 3 are illustrated in FIG. 25.

A stent system is provided in a fifth embodiment of the present disclosure. The stent system includes the stent graft 1 of any implementation described above and an interventional instrument for delivering the stent graft 1 to a target vessel. Reference can be made to FIG. 26, which is a schematic structural view of a stent system in a fifth embodiment (schematic view illustrating at least partial release of a proximal end of the stent graft). The interventional instrument includes an interventional sheath tube 5, a guide head 6, an interventional sheath core 7, and an operation handle (not shown in the figure). The guide head 6 is fixed at a proximal end of the interventional sheath core 7. The interventional sheath tube 5 is sleeved over the interventional sheath core 7. A radial dimension of an inner cavity of the interventional sheath tube 5 is smaller than a radial dimension of a distal end of the guide head 6, so that a proximal end of the interventional sheath tube 5 is in contact with the distal end of the guide head 6, which is conducive to preventing the interventional sheath tube 5 from extending out of the proximal end of the interventional sheath core 7 when the interventional sheath tube 5 moves towards a proximal end of the interventional instrument. There is a gap between the interventional sheath tube 5 and the interventional sheath core 7 for accommodating the radially compressed stent graft 1. It should be noted that the radially compressed stent graft 1 refers to a stent graft 1 whose radial dimension is smaller than the radial dimension of a stent graft 1 after self-expansion and release. The interventional sheath core 7 is configured to detachably secure the proximal end of the stent graft 1, for example, the stent graft 1 is hooked onto the interventional sheath core 7 through at least a part of the crest 1022 at the proximal end of the main support frame 102. The axial length of the stent graft 1 is smaller than the axial length of the interventional sheath core 7, so the stent graft 1 is located at the portion of the interventional sheath core 7 close to the proximal end of the interventional sheath core 7. The operation handle is used for operating axial movement of the interventional sheath tube 5 in the axial direction of the interventional sheath core 7. When the operation handle controls the interventional sheath tube 5 to move axially towards the distal end of the interventional sheath core 7, the stent graft 1 is exposed relative to the interventional sheath tube 5. When the operation handle controls the interventional sheath tube 5 to move axially towards the proximal end of the interventional sheath core 7, and the proximal end of the interventional sheath tube 5 does not exceed the proximal end of the interventional sheath core 7, the interventional sheath tube 5 wraps the stent graft 1 so that the stent graft 1 is limited to the gap between the interventional sheath tube 5 and the interventional sheath core 7.

The release process of the main stent 10 from the interventional sheath tube 5 is typically achieved by withdrawing the interventional sheath tube 5 to release the main stent 10, that is, by withdrawing the interventional sheath tube 5, the main stent 10 is exposed relative to the inner cavity of the interventional sheath tube 5. In the process of withdrawing the interventional sheath tube 5, the proximal end of the main stent 10 is usually released first, and at this time, the first fenestration 111 closer to the proximal end of the main stent 10 is preferentially aligned with the root opening of the corresponding branch vessel 4. Therefore, it can be understood that once the alignment between the first fenestration 111 and the root opening of the corresponding branch vessel 4 is determined, the position of the main stent 10 in the trunk vessel is basically determined. On condition that the distance between the center positions of the two fenestrations 11 is given, the length of the second fenestration 112 in the axial direction of the main stent 10 is set to be larger, so that the edge of the second fenestration 112 is closer to the edge of the first fenestration 111, which is conducive to improving the fault tolerance rate of the second fenestration 112. In this way, when the main stent 10 is released in the trunk vessel, it is unnecessary to align the center line of the second fenestration 112 precisely with the center line of the target branch vessel 4, thereby reducing the requirement for interventional treatment technology of the operator, and reducing the operation time. Meanwhile, by setting the second fenestration 112 to have larger length in the axial direction of the main stent 10 and to be closer to the first fenestration 111, the super-selective positioning is more facilitated. Further, by setting the second fenestration 112 to have larger length in the axial direction of the main stent 10 and to be closer to the first fenestration 111, the wider application range is achieved, so that different distances between two adjacent branch vessels 4 on the trunk vessel of different patients can be adapted. The positioning of the third fenestration 113 follows the same principle.

In some implementations, a pre-embedded guidewire (not shown) is routed through the connecting stent 20, and the pre-embedded guidewire is also routed through the main stent 10, that is, the pre-embedded guidewire is radially compressed together with the main stent 10 and assembled within the interventional sheath tube 5. One end of the pre-embedded guidewire is located outside the connecting stent 20, and the other end of the pre-embedded guidewire is located outside the interventional sheath tube 5. Therefore, after one end of the pre-embedded guidewire located outside the connecting stent 20 is introduced into the corresponding branch vessel 4, it facilitates the release of the branch stent 2 along the pre-embedded guidewire into the corresponding branch vessel 4. The provision of the pre-embedded guidewire also facilitates aligning the fenestration where the connecting stent 20 is located with the root opening of the target branch vessel 4 first. The pre-embedded guidewire is routed through the connecting stent, and the pre-embedded guidewire is also routed through the main stent. One end of the pre-embedded guidewire is located outside the connecting stent, and the other end of the pre-embedded guidewire is located outside the interventional sheath tube.

An application method for a stent graft 1 is provided in a sixth embodiment of the present disclosure. The stent graft 1 is the stent graft 1 of any implementation described above. The at least two fenestrations 11 are defined on the sidewall of the main stent 10. The dimension of the fenestration 11 gradually increases in a direction from the proximal end of the main stent 10 to the distal end of the main stent 10. When the stent graft 1 is implanted, the fenestration 11 with smaller dimension is first aligned with the target branch vessel 4, while the other fenestrations 11 with larger dimension have a high fault tolerance rate of alignment, which facilitates the successful reconstruction of the branch vessel 4 by the stent graft 1.

For any of the above-described implementations, a material of the main covering membrane 101 includes, but is not limited to, at least one of polyethylene terephthalate (PET), polytetrafluoroethylene (PTFE), thermoplastic polyurethanes (TPU), silicone rubber, polyvinyl alcohol (PVA), hydrogel, expanded polytetrafluoroethylene, polyethylene, high-density polyethylene, polyethylene terephthalate, or other polymer materials.

The main support frame 102 and the support ring may be formed from a material with shape memory effect by laser cutting, or may also be woven from a filamentous material with shape memory effect. A material with shape memory effect includes, but is not limited to, at least one of stainless steel, tungsten alloy, cobalt chromium alloy, or nickel titanium alloy.

A material of the radiopaque structure 209 may be a material with good X-ray opacity, strong corrosion resistance, and good biocompatibility, for example, may be a material such as gold, platinum, tantalum, osmium, rhenium, tungsten, iridium, rhodium, or the like, or an alloy thereof. The radiopaque structure 209 may take various forms, including ring-shaped, wire-shaped, strip-shaped, or dot-shaped, and may be fixed to the connecting stent 20 or the anchor stent 30 by suturing, stamping, inlaying, heat-fusing, bonding, welding, or riveting.

The foregoing descriptions are merely specific implementations of the present disclosure, but are not intended to limit the protection scope of the present disclosure. Any variation or replacement readily figured out by a person skilled in the art within the technical scope disclosed in the present disclosure shall fall within the protection scope of the present disclosure. Therefore, the protection scope of the present disclosure shall be subject to the protection scope of the claims.

## Claims

1. A stent graft, comprising:
a main stent, wherein the main stent defines two openings at two ends of the main stent in an axial direction of the main stent, and a lumen extending axially between the two openings, and the main stent defines at least one fenestration on a sidewall of the main stent; and
a connecting stent, wherein the connecting stent defines a first opening, a second opening, and an inner cavity extending between the first opening and the second opening, the first opening and the second opening are defined at two ends of the connecting stent in an axial direction of the connecting stent, and the connecting stent is fixed to the at least one fenestration of the main stent through one end of the connecting stent where the second opening is located, to enable the connecting stent to be in communication with the main stent;
wherein the connecting stent has a first configuration and a second configuration, the connecting stent is switchable between the first configuration and the second configuration, and an axial length of the connecting stent in the first configuration is larger than an axial length of the connecting stent in the second configuration.

2. The stent graft of claim 1, wherein the connecting stent at least partially consists of a corrugated tube in the axial direction of the connecting stent.

3. The stent graft of claim 2, wherein the connecting stent comprises a first segment and a second segment that is fixed to and in communication with the first segment, and at least one of the first segment of the connecting stent or the second segment of the connecting stent consists of the corrugated tube in the axial direction of the connecting stent.

4. The stent graft of any one of claims 1 to 3, wherein the connecting stent in the first configuration is located outside the main stent.

5. The stent graft of any one of claims 1 to 4, wherein the connecting stent in the second configuration is at least partially located in the lumen of the main stent.

6. The stent graft of any one of claims 1 to 5, wherein the connecting stent comprises a first segment and a second segment that is fixed to and in communication with the first segment, and when the connecting stent is in the first configuration, the first segment and the second segment are arranged in the axial direction of the connecting stent, a neck of the connecting stent is formed at a connection of the first segment and the second segment, and a cross-sectional diameter of the neck is smaller than at least one of a cross-sectional diameter of the first opening or a cross-sectional diameter of the second opening.

7. The stent graft of claim 6, wherein when the connecting stent is in the first configuration, the first segment is farther from the fenestration of the main stent than the second segment; and the first opening is located at one end of the first segment away from the second segment, and the second opening is located at one end of the second segment away from the first segment; and
in the axial direction of the connecting stent, the cross-sectional diameter of the first segment gradually decreases from one end of the connecting stent where the first opening is located to the neck, and/or the cross-sectional diameter of the second segment gradually decreases from one end of the connecting stent where the second opening is located to the neck.

8. The stent graft of any one of claims 6 to 7, wherein the connecting stent comprises a connecting covering membrane and at least one support member, the connecting covering membrane forms the first segment and the second segment by the neck, and the at least one support member is fixed to a peripheral wall of the first segment.

9. The stent graft of claim 8, wherein the second segment is a flexible segment composed of the connecting covering membrane.

10. The stent graft of any one of claims 6 to 9, wherein the connecting stent comprises a connecting member, and the connecting member at least partially surrounds and is fixed to the neck.

11. The stent graft of claim 10, wherein the connecting member is at least one of a suture set or a connecting rod, and the suture set is at least one suture fixed by suturing to the neck.

12. The stent graft of any one of claims 1 to 11, wherein the connecting stent in the second configuration is at least partially located in the lumen of the main stent.

13. The stent graft of any one of claims 3 to 12, wherein when the connecting stent is in the second configuration, the second segment is at least partially located in the lumen of the main stent.

14. The stent graft of any one of claims 3 to 13, wherein the first segment is at least partially overlappable with the second segment in the axial direction of the connecting stent, and when the connecting stent is in the second configuration, the second segment and the first segment each are at least partially located in the lumen of the main stent.

15. The stent graft of any one of claims 1 to 14, wherein a cross-sectional diameter of the first opening is larger than, smaller than, or equal to a cross-sectional diameter of the second opening.

16. The stent graft of any one of claims 1 to 15, wherein the stent graft further comprises at least one anchor stent, the at least one fenestration is implemented as at least two fenestrations defined on the sidewall of the main stent, and the at least two fenestrations are a first fenestration and a second fenestration, respectively; the connecting stent and the at least one anchor stent are respectively fixed to the first fenestration and the second fenestration, the connecting stent is in communication with the lumen of the main stent through the first fenestration, and the at least one anchor stent is in communication with the lumen of the main stent through the second fenestration; and the at least one anchor stent is located at at least one of an inside of the lumen of the main stent or an outside of the lumen of the main stent.

17. The stent graft of claim 16, wherein the first fenestration is closer to a proximal end of the main stent than the second fenestration, the connecting stent is fixed to the first fenestration, and the at least one anchor stent is fixed to the second fenestration.

18. The stent graft of claim 17, wherein in the axial direction of the main stent from the proximal end of the main stent to a distal end of the main stent, the first fenestration and the second fenestration are spaced apart or not spaced apart from each other.

19. The stent graft of any one of claims 16 to 18, wherein the at least one anchor stent is named at least one first stent; each of the at least one first stent defines a first opening, a second opening, and an inner cavity extending between the first opening and the second opening, and the first opening and the second opening are defined at two ends of a first stent in an axial direction of the first stent; and the first stent is fixed to the second fenestration through one end of the first stent where the second opening is located, to enable the first stent to be in communication with the main stent; and
the first stent has a first configuration and a second configuration, the first stent is switchable between the first configuration and the second configuration, and an axial length of the first stent in the first configuration is larger than an axial length of the first stent in the second configuration.

20. The stent graft of claim 19, wherein the first stent in the first configuration is located in the lumen of the main stent.

21. The stent graft of claim 19 or 20, wherein the first stent in the second configuration is partially located outside the main stent.

22. The stent graft of any one of claims 19 to 21, wherein the first stent comprises a first segment and a second segment fixed to the first segment, and when the first stent is in the first configuration, the first segment of the first stent and the second segment of the first stent are arranged in the axial direction of the connecting stent, and a neck of the first stent is formed at a connection of the first segment and the second segment; the first opening of the first stent is located at one end of the first segment of the first stent away from the second segment, and the second opening of the first stent is located at one end of the second segment of the first stent away from the first segment; and a cross-sectional dimension of the first stent at the neck is smaller than at least one of a cross-sectional dimension of the first opening or a cross-sectional dimension of the second opening.

23. The stent graft of any one of claims 19 to 22, wherein for each of the at least one first stent, in the axial direction of the first stent, a cross-sectional diameter of the first segment gradually decreases from one end of the first stent where the first opening is located to the neck, and/or a cross-sectional diameter of the second segment gradually decreases from one end of the first stent where the second opening is located to the neck.

24. The stent graft of any one of claims 19 to 23, wherein a cross-sectional dimension of the first opening of the first stent is smaller than, larger than, or equal to a cross-sectional dimension of the second opening of the first stent.

25. The stent graft of any one of claims 19 to 24, wherein the first stent comprises a connecting covering membrane and at least one support member, the connecting covering membrane of the first stent forms the first segment and the second segment by the neck, and the at least one support member is fixed to a peripheral wall of the first segment of the first stent.

26. The stent graft of claim 25, wherein at least a part of the second segment of the first stent is a flexible segment composed of the connecting covering membrane.

27. The stent graft of any one of claims 19 to 26, wherein when the first stent is in the second configuration, the second segment is at least partially located outside the lumen of the main stent.

28. The stent graft of any one of claims 19 to 27, wherein the first segment of the first stent is at least partially overlappable with the second segment of the first stent in the axial direction of the first stent, and when the first stent is in the second configuration, the second segment and the first segment each are at least partially located outside the lumen of the main stent.

29. The stent graft of any one of claims 19 to 28, wherein the axial length of the connecting stent in the first configuration is larger than the axial length of the first stent in the first configuration.

30. The stent graft of any one of claims 1 to 29, wherein the stent graft further comprises at least two anchor stents, the at least one fenestration is implemented as at least three fenestrations defined on the sidewall of the main stent, and the at least three fenestrations are a first fenestration, a second fenestration, and a third fenestration, respectively;
the connecting stent and the at least two anchor stents are fixed to the first fenestration, the second fenestration, and the third fenestration, respectively, the connecting stent is in communication with the lumen of the main stent through the first fenestration, at least one of the at least two anchor stents is in communication with the lumen of the main stent through the second fenestration, and at least one of the at least two anchor stents is in communication with the lumen of the main stent through the third fenestration; and
any one of the at least two anchor stents is located in the lumen of the main stent or outside the lumen of the main stent.

31. The stent graft of claim 30, wherein the first fenestration is closer to a proximal end of the main stent than the second fenestration, and the second fenestration is closer to the proximal end of the main stent than the third fenestration; and the connecting stent is fixed to the first fenestration, at least one of the at least two anchor stents is fixed to the second fenestration, and at least one of the at least two anchor stents is fixed to the third fenestration.

32. The stent graft of claim 31, wherein in the axial direction of the main stent from the proximal end of the main stent to a distal end of the main stent, the first fenestration, the second fenestration, and the third fenestration are spaced apart or not spaced apart from one another.

33. The stent graft of any one of claims 1 to 29, wherein the stent graft comprises at least one anchor stent, the connecting stent is implemented as at least two connecting stents, the at least one fenestration is implemented as at least three fenestrations defined on the sidewall of the main stent, and the at least three fenestrations are a first fenestration, a second fenestration, and a third fenestration, respectively;
the at least two connecting stents and at least one anchor stent are fixed to the first fenestration, the second fenestration, and the third fenestration, respectively, at least one of the at least two connecting stents is in communication with the lumen of the main stent through the first fenestration, at least one of the at least two connecting stents is in communication with the lumen of the main stent through the second fenestration, and the at least one anchor stent is in communication with the lumen of the main stent through the third fenestration; and
at least one of the at least two connecting stents in the first configuration is located outside or in the lumen of the main stent.

34. The stent graft of any one of claims 1 to 18, wherein the connecting stent is implemented as at least three connecting stents, the at least one fenestration is implemented as at least three fenestrations defined on the sidewall of the main stent, the at least three connecting stents are respectively fixed to the at least three fenestrations, and the at least three connecting stents are respectively in communication with the lumen of the main stent through the at least three fenestrations fixed thereby; and at least one of the at least three connecting stents in the first configuration is located outside or in the lumen of the main stent.

35. The stent graft of any one of claims 1 to 34, wherein at least one of two ports of the at least one connecting stent in the axial direction of the connecting stent is configured as an oblique port.

36. The stent graft of any one of claims 16 to 33, wherein at least one of two ports of each of the at least one anchor stent in an axial direction of an anchor stent is configured as an oblique port.

37. The stent graft of any one of claims 16 to 36, wherein the at least two fenestrations are spaced apart or not spaced apart from each other in a direction from a proximal end of the main stent to a distal end of the main stent; and projections of the at least two fenestrations at least partially overlap in the axial direction of the main stent, and/or central axes of the at least two fenestrations coincide.

38. The stent graft of any one of claims 16 to 36, wherein the at least three fenestrations are spaced apart or not spaced apart from one another in a direction from a proximal end of the main stent to a distal end of the main stent; and projections of the at least three fenestrations at least partially or completely overlap in the axial direction of the main stent, and/or central axes of the at least three fenestrations coincide.

39. The stent graft of any one of claims 16 to 38, wherein in a direction from a proximal end of the main stent to a distal end of the main stent, lengths of the plurality of fenestrations in the axial direction of the main stent sequentially increase.

40. The stent graft of any one of claims 16 to 39, wherein in a direction from a proximal end of the main stent to a distal end of the main stent, lengths of the plurality of fenestrations in a circumferential direction of the main stent sequentially increase.

41. The stent graft of any one of claims 16 to 40, wherein the at least two fenestrations are defined on the sidewall of the main stent, and the at least two fenestrations are the first fenestration and the second fenestration, respectively, the first fenestration is closer to a proximal end of the main stent than the second fenestration, and a length of the second fenestration in a circumferential direction of the main stent is at least 50%-70% of a diameter of the main stent.

42. The stent graft of any one of claims 16 to 40, wherein the at least two fenestrations are implemented as at least three fenestrations defined on the sidewall of the main stent, the at least three fenestrations are a first fenestration, a second fenestration, and a third fenestration, respectively, the first fenestration is closer to a proximal end of the main stent than the second fenestration, and the second fenestration is closer to the proximal end of the main stent than the third fenestration; and a length of the second fenestration in a circumferential direction of the main stent is at least 50%-70% of a diameter of the main stent, and/or a length of the third fenestration in the circumferential direction of the main stent is at least 60%-90% of the diameter of the main stent.

43. The stent graft of any one of claims 1 to 42, wherein the main stent comprises a main covering membrane and a plurality of main support frames fixed to the main covering membrane, the plurality of main support frames are spaced apart in an axial direction of the main covering membrane; and each of the plurality of main support frames comprises a plurality of support rods sequentially connected at angles, two adjacent angles in a circumferential direction of the main stent are respectively defined as a crest and a trough, and the crest is closer to a proximal end of the main stent than the trough; and
for a main support frame of the plurality of main support frames at at least one side of each of the at least one fenestration in the axial direction of the main stent, two adjacent support rods of the main support frame in the circumferential direction of the main stent define a surrounding angle therebetween, the surrounding angle and the two adjacent support rods defining the surrounding angle cooperatively form a semi-annular structure, and the semi-annular structure at least partially surrounds a fenestration.

44. The stent graft of claim 43, wherein the semi-annular structure is at least partially raised in a direction away from a central axis of the main stent.

45. The stent graft of claim 43 or 44, wherein the surrounding angle is raised in a direction away from a central axis of the main stent compared with other angles on the main support frame where the surrounding angle is located, or the surrounding angle and at least a part of each of the two support rods defining the surrounding angle are raised.

46. The stent graft of any one of claims 1 to 45, wherein the main stent comprises a first support segment, a recessed segment, and a second support segment extending sequentially from a proximal end of the main stent to a distal end of the main stent, a radial dimension of the first support segment is larger than each of a radial dimension of the recessed segment and a radial dimension of the second support segment, and the radial dimension of the recessed segment is smaller than the radial dimension of the second support segment, to enable the recessed segment to be at least partially recessed in a radial direction of the main stent.

47. The stent graft of claim 46, wherein at least two fenestrations are located on the recessed segment; or the at least one fenestration is located on the first support segment.

48. The stent graft of claim 46 or 47, wherein the recessed segment comprises an avoidance segment and at least one flared portion; the at least one flared portion extends from a proximal end of the avoidance segment towards the proximal end of the main stent, and the recessed segment is fixed to a distal end of the first support segment through a proximal end of the flared portion; and in a circumferential direction of each of the at least one flared portion, each of the at least one flared portion is at least partially radially enlarged in a direction away from a central axis of the recessed segment.

49. The stent graft of claim 48, wherein one of the at least one fenestration close to the proximal end of the main stent is located on one of the at least one flared portion close to the proximal end of the main stent; or each of the at least one fenestration is located on the avoidance segment.

50. A stent system comprising a branch stent and the stent graft of any one of claims 1 to 49, wherein the branch stent is insertable into the connecting stent through either of the first opening of the connecting stent and the second opening of the connecting stent; and/or the branch stent is insertable into an anchor stent of the stent graft.

51. A stent system comprising the stent graft of any one of claims 1 to 49 and an interventional instrument for delivering the stent graft to a target position, wherein the interventional instrument comprises a guide head, an interventional sheath core fixed at a distal end of the guide head, an interventional sheath tube sleeved over the interventional sheath core, and an operation handle for controlling axial movement of the interventional sheath tube, a gap is defined between the interventional sheath tube and the interventional sheath core for accommodating the stent graft that is radially compressed; and a proximal end of the stent graft is detachably fixed to the interventional sheath core.

52. The stent system of claim 51, wherein a pre-embedded guidewire is routed through the connecting stent, the pre-embedded guidewire is also routed through the main stent, one end of the pre-embedded guidewire is located outside the connecting stent, and another end of the pre-embedded guidewire is located outside the interventional sheath tube.

53. An application method for a stent graft, applicable to the stent graft of any one of claims 16 to 49, and the application method comprises:
aligning the first fenestration or the second fenestration with a target branch vessel first and then aligning other fenestrations with the target branch vessel, when the stent graft is implanted.
